# EUROPEAN PATENT APPLICATION

(11) **EP 1 864 985 A1**
(43) Date of publication of application: **12.12.2007**
(21) Application number: 06729744.0
(22) Date of filing: 23.03.2006
(51) Int. Cl.: C07D 477/14, A61K 31/407, A61K 31/4178, A61K 31/422, A61K 31/5377, A61P 31/04

(54) **NOVEL CARBAPENEM COMPOUND**

(30) Priority: 25.03.2005 JP 2005088672
(71) Applicant: Dainippon Sumitomo Pharma Co., Ltd., Osaka 541-8524 (JP)
(72) Inventor: TSUKIMURA, T., Oita-shi, Oita, 8700106 (JP); SASAKI, Akira, Takarazuka-shi, Hyogo 665-0805 (JP); SUNAGAWA, Makoto, Itami-shi, Hyogo 664-0875 (JP)
(74) Representative: Vossius & Partner
(86) International application number: PCT/JP2006/305777
(87) International publication number: WO 2006/103999

(57) **Abstract**

An orally administrable antibacterial agent which contains as an active ingredient a carbapenem compound represented by the formula [1] below, 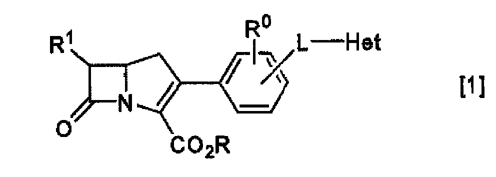 wherein R⁰ represents hydrogen atom or the like; R¹ represents C₁-C₃ alkyl substituted by hydroxyl group or the like; R represents hydrogen atom or a group which regenerates a carboxyl group by hydrolysis in a living body; L represents a single bond, methylene, -OCH₂(CO)- or the like; and Het represents a group represented by the following formula [2], 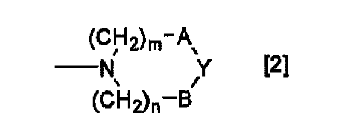 wherein m and n independently represent 0 or 1; A and B independently represent methylene, carbonyl or the like; Y represents methylene, ethylene, oxygen atom, -OCH₂-, -NR^{a}CH₂- (wherein R^{a} represents hydrogen atom, optionally substituted C₁-C₄ alkyl group or the like) or the like.

## Description

### TECHNICAL FIELD

The present invention relates to a novel carbapenem compound. In more detail, the present invention relates to a carbapenem compound, wherein a phenyl with a heterocyclic ring via a spacer is directly substituted at position 3 of 7-oxo-1-azabicyclo[3.2.0]hept-2-ene which is a basic nucleus of the carbapenem compound. Furthermore, the present invention relates to an antibacterial agent containing such a compound as an active ingredient.

### BACKGROUND ART

The carbapenem compounds which have been developed and commercialized are poor in absorbability from the digestive tract and therefore, they are clinically used only in the form of injection, mainly intravenous injection. However, in the clinical field, it is desirable to select several administration routes from the viewpoint of circumstances or wishes of a patient, a therapeutic object, etc. Especially, oral administration of an antibacterial agent is easy and convenient for administration to a patient in comparison with injection. In view of the care of a patient at home, oral administration of the antibacterial agent is more convenient and the clinical usability is extremely high. It has been strongly desired in the clinical field to develop a carbapenem compound which has a potent antibacterial activity especially against Haemophilus influenzae which widely gain resistance to the inhibitory effect of existing β-lactam agents together with mutation of a penicillin binding proteins (PBP), such as β-lactamase non-producing ampicillin resistant Haemophilus influenzae (BLNAR), and penicillin resistant Streptococcus pneumoniae (PRSP) which have been recently increasingly isolated and provide a clinical trouble, and is rich in safety and is orally administrable. However none of such agents has been put on the market. Orally administrable tricyclic carbapenem compounds which have been studied and developed until now are disclosed for example, in WO 92/03437. These compounds have a characteristic structure in a side chain having a ring which is fused via C-C bond and they are modified to a prodrug thereof for increase of oral absorbability, but their safety in the clinical test is not reported. Besides, there are several known 1β methylcarbapenem compounds (See Japanese patent 2-49783A, Japanese patent 8-53453A, Japanese patent 4-279588A, Japanese patent 2-223587A, WO 98/34936, WO 99/57121, Antimicrobial Agents and Chemotherapy, Mar. 1999, p460-464). All of them have a structural property having 1β-methyl group and a side chain via sulfide bond which are said to contribute to an increase of chemical stability and in a living body (biological) stability, and are modified to a prodrug of them for increase of oral absorbability. Especially, the clinical trials are carried out on compounds disclosed in Japanese patent 2-49783A and Japanese patent 8-53453A, but the safety of them and so on have been not clear.

On the other hand, carbapenem compounds having an aryl ring via C-C bond as a side chain structure were known since 1980s (See US Patent 4543257, US Patent 4775669, US Patent 5258509, Tetrahedron, 1983, Vol. 39, p2531-2549, and Journal of Medicinal Chemistry, 1987, Vol. 30, p871-880), and carbapenem compounds directly substituted by a phenyl with a hetero ring via a spacer in the structure are also known (See EP 0414493 and EP 0465126). These reports are concerned only to studies and developments on injections thereof, but the Drug-Approval for oral application on them has not been done.

Although WO 02/053566, WO 03/040146, WO 03/089431 and WO 2004/089954 disclose orally administrable carbapenem compounds with an aryl ring, but carbapenem derivatives having above substituents of the present invention are novel.

### DISCLOSURE OF INVENTION

The object of the present invention is to provide a carbapenem compound which has a potent antibacterial activity against Gram positive bacteria and Gram negative bacteria, especially Haemophilus influenzae which obtain resistance to the inhibitory effect of existing β-lactam agents together with mutation of a penicillin binding proteins (PBP) such as β-lactamase non-producing ampicillin resistant Haemophilus influenzae (BLNAR), and penicillin resistant Streptococcus pneumoniae (PRSP) which are recently increasingly isolated and provide a clinical problem, and has excellent oral absorbability.

The present inventors have intensively studied to find that the carbapenem compound, wherein a phenyl with a heterocyclic ring via a spacer is directly substituted at position 3 of 7-oxo-1-azabicyclo[3.2.0]hept-2-ene which is a basic nucleus of the carbapenem compound, has a broad spectrum and a potent antibacterial activity against Gram positive bacteria and Gram negative bacteria, especially against Haemophilus influenzae which obtain resistance to inhibitory effect of existing β-lactam agents together with mutation of a penicillin binding proteins (PBP) such as β-lactamase non-producing ampicillin resistant Haemophilus influenzae (BLNAR), and penicillin resistant Streptococcus pneumoniae (PRSP) which have been recently increasingly isolated and provide a clinical problem. Further, they have also found that a compound having a group substituted onto the 2-carboxyl group, the said group being capable of regenerating a carboxyl group by hydrolyzing in the living body, shows a good absorbability from the digestive tract by oral administration, and shows a potent antibacterial activity after converted into a 2-de-esterified compound in the living body, and further shows an excellent resistance to renal dehydropeptidase, and finally have accomplished the present invention.

Namely, the present invention relates to:
(1) a carbapenem compound represented by the formula [1], 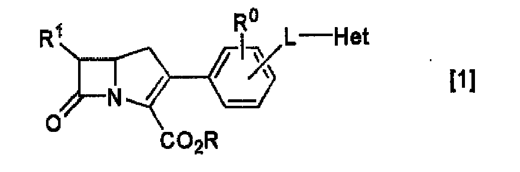 wherein R⁰ is hydrogen atom, C₁ to C₄ alkyl, C₁ to C₄ alkoxy, trifluoromethoxy, halogen atom or cyano group,
   R¹ is C₁ to C₃ alkyl or C₁ to C₃ alkyl substituted by hydroxy,
   R is hydrogen atom or a group which regenerates a carboxyl group by hydrolysis in a living body,
   L is a single bond, methylene, ethylene, propylene, -O(CH₂)₂- or -OCH₂(CO)-, and
   Het is a monocyclic heteroaromatic ring having a nitrogen atom(s), or a group represented by the formula [2]: 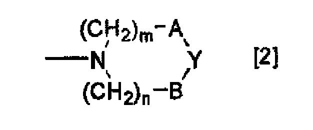 wherein m and n are independently 0 or 1,
   A and B are independently methylene, carbonyl or thiocarbonyl,
   Y is methylene, ethylene, oxygen atom, -OCH₂-, sulfur atom, -SCH₂-, -NR^{a}- or -NR^{a}CH₂- (wherein R^{a} is hydrogen atom, amine protective group or optionally substituted C₁ to C₄ alkyl group), provided that when Y is methylene, either A or B is at least carbonyl or thiocarbonyl, and when m and n are 0, and A and B are independently carbonyl or thiocarbonyl, Y is methylene, ethylene or propylene,
   or its pharmaceutically acceptable salt;
(2) a carbapenem compound according to the above (1) wherein the group which regenerates a carboxyl group by hydrolysis in a living body is a group of the formula [3], 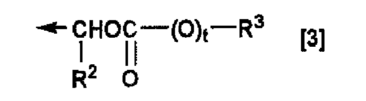 wherein R² is hydrogen atom or C₁ to C₆ alkyl group,
   R³ is an optionally substituted C₁ to C₁₀ alkyl group, or an optionally substituted C₃ to C₁₀ cycloalkyl group,
   t is 0 or 1,
   or (5-t-butyl-2-oxo-1,3-dioxol-4-yl)methyl,
   or its pharmaceutically acceptable salt;
(3) a carbapenem compound according to the above (1) wherein R is a group represented by the formula [3], 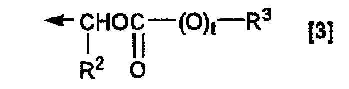 wherein R², R³ and t are the same as in above (2),
   or (5-t-butyl-2-oxo-1,3-dioxol-4-yl)methyl,
   or its pharmaceutically acceptable salt;
(4) a carbapenem compound according to any one of the above (1) to (3) wherein R¹ is 1-hydroxyethyl, or its pharmaceutically acceptable salt;
(5) a carbapenem compound according to any one of the above (1) to (4) wherein Het is morpholino, or its pharmaceutically acceptable salt;
(6) a carbapenem compound according to any one of the above (1) to (4) wherein Het is a group of the formula [4], 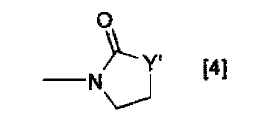 wherein Y' is methylene, ethylene, oxygen atom, -OCH₂-, -CH₂O-, sulfur atom, -SCH₂-, -CH₂S-, -NR^{a}-, -NR^{a}CH₂- or -CH₂NR^{a}- (wherein R^{a} is hydrogen atom, amino protecting group or optionally substituted C₁ to C₄ alkyl group), or its pharmaceutically acceptable salt;
(7) a carbapenem compound according to any one of the above (1) to (6) wherein L is a single bond, ethylene, propylene or -O(CH₂)₂-, or its pharmaceutically acceptable salt;
(8) a carbapenem compound according to any one of the above (1) to (6) wherein L is methylene, or its pharmaceutically acceptable salt;
(9) a carbapenem compound according to any one of the above (1) to (3) wherein R is pivaloyloxymethyl or (5-t-butyl-2-oxo-l,3-dioxol-4-yl)methyl, or its pharmaceutically acceptable salt;
(10) a carbapenem compound according to any one of the above (1) to (9) wherein Het is a monocyclic heteroaromatic ring containing a nitrogen atom(s), or its pharmaceutically acceptable salt;
(11) a medicament containing the carbapenem compound or its pharmaceutically acceptable salt according to any one of the above (1) to (10) as an active ingredient; and
(12) an antibacterial agent for oral administration containing the carbapenem compound or its pharmaceutically acceptable salt according to any one of the above (1) to (10) as an active ingredient.

According to the present invention it becomes possible to provide a carbapenem compound which has a broad spectrum and a potent antibacterial activity against Gram positive bacteria and Gram negative bacteria, especially against Haemophilus influenzae which obtain resistance to the inhibitory effect of existing β-lactam agents together with mutation of a penicillin binding proteins (PBP) such as β-lactamase non-producing ampicillin resistant Haemophilus influenzae (BLNAR), and penicillin resistant Streptococcus pneumoniae (PRSP) which are recently increasingly isolated and provide a clinical problem, and is rich in safety and has excellent oral absorbability.

### BEST MODE FOR CARRYING OUT THE INVENTION

The first aspect of the present invention relates to the above carbapenem compounds. Various terms and preferable examples referred to the present specification are explained as follows.

"C₁ to C₄ alkyl" in R⁰ and R^{a} includes, for example methyl, ethyl, n-propyl, isopropyl, n-butyl, isobutyl, or tert-butyl, preferably for example methyl, ethyl, n-propyl, or isopropyl, especially preferably methyl or ethyl. The substituent of "C₁ to C₄ alkyl group optionally substituted" in R^{a}, includes hydroxy group, C₁ to C₄ alkoxy group such as methoxy group or ethoxy group, C₁ to C₄ alkylthio group such as methylthio or ethylthio, C₂ to C₅ alkanoyl group such as acetyl or propionyl, C₂ to C₅ alkanoyloxy group such as acetoxy or propionyloxy, C₂ to C₅ alkoxycarbonyl group such as methoxycarbonyl or ethoxycarbonyl, carboxyl group, fluorine atom, chlorine atom, bromine atom, iodine atom, cyano group, amino group, mono or di(C₁ to C₄ alkyl) amino group such as methylamino or dimethylamino, aminocarbonyl group, mono or di(C₁ to C₄ alkyl)aminocarbonyl group such as methylaminocarbonyl or dimethylaminocarbonyl, aminocarbonyloxy, mono or di(C₁ to C₄ a1kyl)arninocarbonyloxy group such as methylaminocarbonyloxy or dimethylaminocarbonyloxy.

"C₁ to C₄ alkoxy" in R⁰ includes methoxy, ethoxy, n-propoxy, isopropoxy, n-butoxy, isobutoxy, sec-butoxy or tert-butoxy, preferably methoxy, ethoxy, n-propoxy or isopropoxy, more preferably methoxy or ethoxy.

"A halogen atom" in R⁰ includes fluorine atom, chlorine atom, bromine atom or iodine atom, preferably fluorine atom or chlorine atom.

"C₁ to C₃ alkyl" in R¹ includes a straight or branched chain C₁ to C₃ alkyl, such as methyl, ethyl, n-propyl, isopropyl, etc., preferably ethyl or isopropyl.

"C₁ to C₃ alkyl substituted by hydroxy" in R¹ includes a group having C₁ to C₃, such as hydroxymethyl, 1-hydroxyethyl, 2-hydroxyethyl, 1-hydroxy-1-methylethyl, 1-hydroxypropyl, etc., preferably 1-hydroxyethyl, 2-hydroxyethyl, 1-hydroxy-1-methylethyl, and more preferably 1-hydroxyethyl.

"A group which regenerates a carboxyl group by hydrolysis in a living body" includes any group as long as the group regenerates a carboxyl group by hydrolysis in a living body, and includes any group which is used for conversion into a compound called a prodrug, preferably a group represented by a following formula [3], 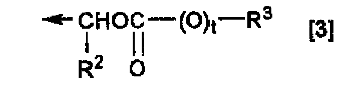 wherein R² is hydrogen atom or C₁ to C₆ alkyl group, R³ is optionally substituted C₁ to C₁₀ alkyl or optionally substituted C₃ to C₁₀ cycloalkyl, and t is 0 or 1.

"C₁ to C₆ alkyl" in R² includes C₁ to C₆ straight or branched alkyl such as methyl, ethyl, n-propyl, isobutyl, tert-butyl, n-pentyl or n-hexyl, preferably methyl.

"C₁ to C₁₀ alkyl" in R³ includes C₁ to C₁₀ straight or branched alkyl such as methyl, ethyl, n-propyl, isobutyl, tert-butyl, n-pentyl, n-hexyl, n-heptyl, n-octyl, n-nonyl, n-decyl, etc., preferably methyl, ethyl, n-propyl, isobutyl, tert-butyl, n-pentyl or n-hexyl.

"C₃ to C₁₀ cycloalkyl" in R³ includes cyclopropyl, cyclobutyl, cyclopentyl, cyclohexyl, cycloheptyl, cyclooctyl, cyclononyl, cyclodecyl, etc., preferably cyclopropyl, cyclobutyl, cyclopentyl or cyclohexyl.

The substituent of "optionally substituted C₁ to C₁₀ alkyl", or "optionally substituted C₃ to C₁₀ cycloalkyl" in R³ includes, for example C₁ to C₆ straight or branched alkyl such as, methyl, ethyl, n-propyl, isobutyl, tert-butyl, n-pentyl, n-hexyl, etc., preferably methyl or ethyl. Namely the group represented by the formula [3] includes preferably, pivaloyloxymethyl, acetyloxymethyl, cyclohexylacetyloxymethyl, 1-methylcyclohexylcarbonyloxymethyl, ethoxycarbonyloxy-1-ethyl, cyclohexyloxycarbonyloxy-1-ethyl, etc., especially preferably, pivaloyloxymethyl. Other examples of "a group which regenerates a carboxyl group by hydrolysis in a living body" include C₁ to C₆ alkyl such as methyl, ethyl, etc., C₂ to C₁₂ alkyloxyalkyl such as methoxymethyl, ethoxymethyl, 2-methoxyethyl, 2-methoxyethoxymethyl, etc., phthalidyl, further (2-oxo-1,3-dioxol-4-yl) methyl, (5-methyl-2-oxo-1,3-dioxol-4-yl)methyl, (5-t-butyl-2-oxo-1,3-dioxol-4-yl)methyl, (5-phenyl-2-oxo-1,3-dioxol-4-yl)methyl, etc, more preferably phthalidyl or (5-methyl-2-oxo-1,3-dioxol-4-yl)methyl.

The protection group of amino group can includes various protecting group used in usual methods, preferably, C₂ to C₇ alkyloxycarbonyl such as tert-butoxycarbonyl, etc., C₁ to C₅ halogenoalkyloxycarbonyl such as 2-iodoethoxycarbonyl, 2,2,2-trichloroethoxycarbonyl, etc., optionally substituted C₂ to C₇ alkenyloxycarbonyl such as allyloxycarbonyl, etc., aralkyloxycarbonyl such as benzyloxycarbonyl, p-methoxybenzyloxycarbonyl, o-nitrobenzyloxycarbonyl, p-nitraben.zylaxycarbonyl, etc., trialkylsilyl such as trimethylsilyl, triethylsilyl, tert-butyldimethylsilyl, etc. Various protective groups which regenerate hydroxy group, amino group and/or amide group by hydrolysis in a living body can be used. The examples thereof are (5-methyl-2-oxo-1,3-dioxal-4-yl)methyl, (5-methyl-2-oxo-1,3-dioxol-4-yl)methyloxycarbonyl, etc.

The monocyclic heteroaromatic ring having a nitrogen atom(s) means a 5 to 6 membered monocyclic heteroaromatic ring containing 1 to 4 hetero atoms selected from 1 to 4 nitrogen atoms, 0 to 2 oxygen atoms and 0 to 2 sulfur atoms and the binding site is limited to at the nitrogen atom. The examples are a five membered heteroaromatic ring such as pyrrole, imidazole, pyrazole, thiazole, oxazole, isothiazole, isoxazole, triazole, etc., and a six membered heteroaromatic ring such as pyridine, pyrimidine, pyrazine, pyridazine, triazine, etc.

The pharmaceutically acceptable salt of the carbapenem of the present invention includes a conventional non-toxic salt. Such salts include, as a salt with an intramolecular carboxylic acid, a salt with an inorganic base, such as sodium, potassium, calcium, magnesium, ammonium salt, etc., a salt with an organic base, such as triethylammonium, pyridinium, diisopropylammonium salt, etc., or as a salt with an intramolecular basic group, a salt with an inorganic acid such as hydrochloric acid, sulfuric acid, phosphoric acid, or a salt with an organic acid, such as formic acid, acetic acid, oxalic acid, methanesulfonic acid, benzenesulfonic acid, etc.

The carbapenem compound of the present invention or the pharmaceutically acceptable salt thereof may be in the form of an anhydride thereof, a hydrate thereof, or a solvate thereof.

The second aspect of the present invention relates to a pharmaceutical composition containing the carbapenem compound of the present invention as an active ingredient.

Since the carbapenem compound of the present invention has a potent antibacterial activity, excellent oral absorbability and furthermore, has excellent stability to DHP-I, the compound is expected as a potent antibacterial agent, especially the agent for oral application, which is clinically applicable.

The carbapenem compound of the present invention exhibits broad antibacterial spectrum including gram positive bacteria, such as Staphylococcus aureus, Staphylococcus epidermidis, Streptococcus pyogenes, Streptococcus pneumoniae, Enterococcus faecalis, etc., and gram negative bacteria, such as Escherichia coli, the genus Proteus, Klebsiella pneumoniae, Haemophilus influenzae, Neisseria gonorrhoe, the genus Branhamella, etc. The carbapenem compound of the present invention has been found to have a potent antibacterial activity especially against Haemophilus influenzae which widely gain resistance to the inhibitory effect of existing β-lactam agents together with mutation of a penicillin binding proteins (PBP), such as β-lactamase non-producing ampicillin resistant Haemophilus influenzae (BLNAR), and penicillin resistant Streptococcus pneumoniae (PRSP) which have been recently increasingly isolated and provide a clinical trouble.

It is well known that dehydropeptidase-I (DHP-I), a renal enzyme can easily hydrolyze a carbapenem derived from natural sources. However, the carbapenem compounds of the present invention is stable to DHP-I and it is possible to use it solely. However, it is possible to use the compound of the present invention together with a DHP-I inhibitor, if necessary.

When used as an antibacterial agent in the treatment of infectious diseases caused by bacteria, the carbapenem compounds of the present invention are administered, for example, orally in the form of a tablet, a capsule, powders, syrup, etc., or parenterally such as intravenous injection, intramuscular injection, or intrarectal administration.

The suitable administration forms as mentioned above may be prepared in a conventional manner by mixing an active ingredient with a pharmaceutically acceptable carrier, excipient, binder, stabilizer, etc. When administered in the form of injection, a pharmaceutically acceptable buffering agent, a solubilizer, an isotonic agent, etc. may be added thereto.

The dosage of the compound varies according to the symptoms, ages, body weights, the administration form, the frequency of the administration, etc., but it is usually in the range of 100 to 3000mg per day for an adult, which is administered once or divided into several dosage units. Besides, the dosage of the compound may be increased or decreased, if necessary.

The carbapenem compound of the present invention is prepared by various known methods (See Tetrahedron, 39, 2531-2549 (1983), Tetrahedron Letters, 31, 2853-2856 (1990), ibid. 34, 3211-3214 (1993), ibid. 36, 4563-4566 (1995), Japanese patent 4-40357B, EP0414493, EP0465126, WO 02/053566, WO03/040146, WO03/089431, etc.). One of these methods, for example is illustrated as follows: 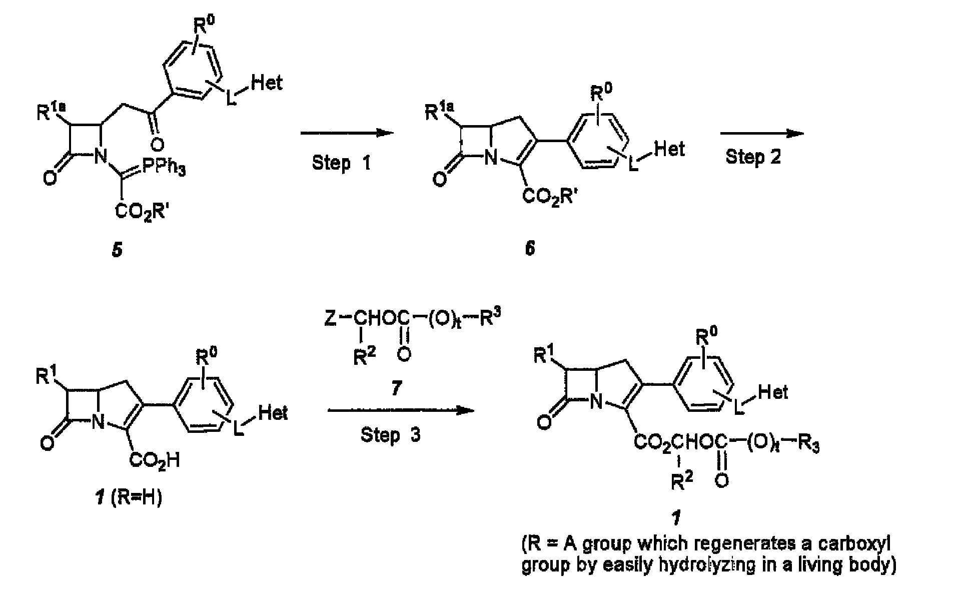 wherein R⁰, R¹, R², R³, L and t are the same as in defined above, R' is a carboxy protecting group, R^{1a} is C ₁ to C ₃ alkyl group or a C ₁ to C ₃ alkyl group substituted by protected hydroxy group, and Z is chlorine atom, bromine atom or iodine atom.

### Step 1: Preparation for compound 6

Compound 5 is cyclized in an inert solvent such as benzene, toluene, xylene at +80°C~200°C to give compound 4. Phosphonium ylide compound 5, starting material can be prepared by known methods such as described in EP0414493, EP0465126, WO02/053566, WO03/040146, WO03/089431, etc.

### Step 2: Preparation of carbapenem compound 1 (R = hydrogen atom)

By removing carboxy protecting group in R' of compound 6, and when R^{1a} is protected hydroxy group, by removing said protecting group, carbapenem compound 1 can be obtained. The removing method for the protecting group is carrying out with a known method, treatment such as with acid, base or hydrolyzing agent (See T.W.Greene, P.G.M.Wuts: Protective Groups in Organic Synthesis; 3rd edition, Wiley, New York (1999) or P.Kocienski, Protecting Groups, Thieme, Stuttgart (1994)).

### Step 3: Preparation for carbapenem compound 1 (R = a group which regenerates a. carboxyl group by hydrolysis in a living body)

By introducing a group which regenerates a carboxyl group by hydrolysis in a living body to a carbapenem compound 1 according to an usual method, a carbapenem compound 1 (R = a group which regenerates a carboxyl group by hydrolysis in a living body) can be obtained. For example, a carbapenem compound 1 (R is hydrogen atom) or its carboxylic acid salt is esterified by reacting various halide compounds represented by compound 7, if necessary in a presence of a base such as diisopropylethylamine, triethyamine, 4-dimethylaminopyridine, potassium carbonate, sodium hydrogen carbonate, etc., or a phase-transfer catalyst such as triethylbenzylammonium chloride, tetrabutylammonium bromide, etc. to give a carbapenem 1 (R = a group which regenerates a carboxyl group by hydrolysis in a living body). The reaction solvent is not limited as long as it is inert, preferably dimethylformamide, dimethyl sulfoxide, hexamethylphosphoramide, acetonitrile, dioxane, tetrahydrofuran, acetone, etc. As carboxylic acid salts there are preferably illustrated, sodium salt, potassium salt, etc. The reaction temperature is -78°C~+100°C, preferably -20°C~+60°C.

When the above reaction is completed, the reacted product is isolated by a conventional organic procedure, but when a water soluble product is obtained, a solution of the reaction mixture is neutralized, and the solution is subjected to a column chromatography using absorption resin, etc., and parts which an object compound is eluted are separated and lyophilized to give the reacted product.

The processes for preparing carbapenem compounds of the present invention are not limited by the above methods.

The optical isomers based on asymmetric carbon atoms on the carbapenem compound of the present invention at the 5- and 6- positions of 7-oxo-1-azabicyclo[3.2.0]hept-2-ene, a basic nuclear, present as shown in a following formula [1],

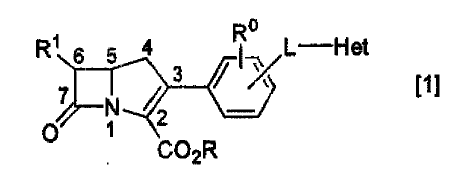

These isomers are all conveniently expressed by only one formula, but the scope of the present invention should not be construed to be limited thereto, and includes all isomers and a mixture of isomers based on each asymmetric carbon atom. The preferable isomers are ones wherein the 5-carbon atom has an R-configuration such as (5R,6R)-compounds or (5R,6S)-compounds. More preferable compounds are ones represented by a following formula [1b],

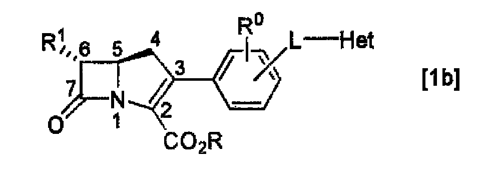

Furthermore, when R¹ is 1-hydroxyethyl group, there are isomers having an R-configuration and an S-configuration at the position 8 as shown in a following formula [1c], and an isomer having the R-confguration is preferable.

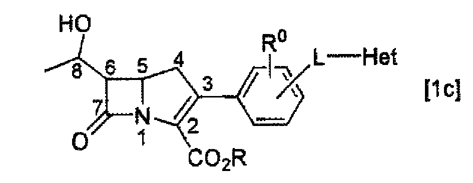

Carbapenem compounds of the present invention are illustrated by compounds 1 to 48.

**Table 1**

| | | |
|---|---|---|
| 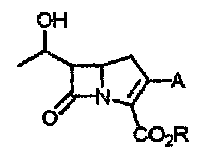 | | |

| Compound No. | R | A |
|---|---|---|
| 1 | -CH₂OCOt-Bu | 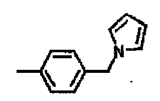 |
| 2 | -CH₂OAc | 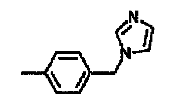 |
| 3 | 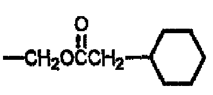 | 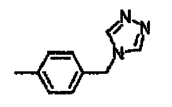 |
| 4 | 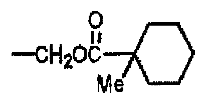 | 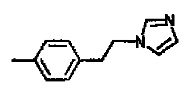 |
| 5 | 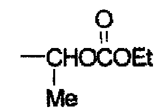 | 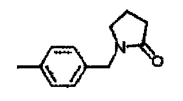 |
| 6 | 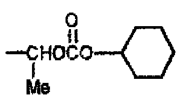 | 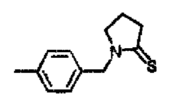 |
| 7 | 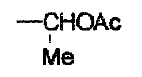 | 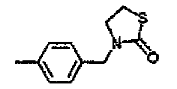 |
| 8 | -CH₂OCOt-Bu | 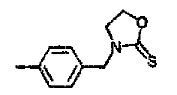 |

**Table 2**

| | | |
|---|---|---|
| 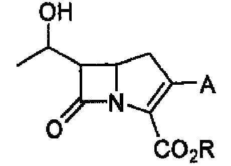 | | |

| Compound No. | R | A |
|---|---|---|
| 9 | -CH₂OCO t-Bu | 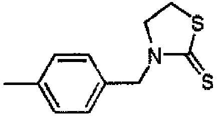 |
| 10 | -CH₂OCO t-Bu | 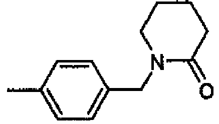 |
| 11 | -CH₂OCO t-Bu | 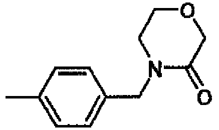 |
| 12 | -CH₂OCO t-Bu | 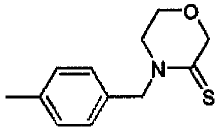 |
| 13 | -CH₂OCO t-Bu | 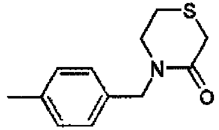 |
| 14 | -CH₂OCO t-Bu | 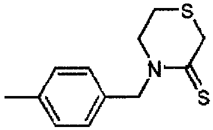 |
| 15 | 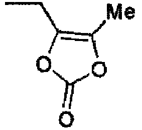 | 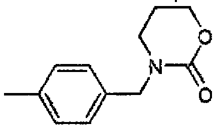 |
| 16 | 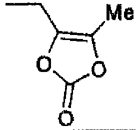 | 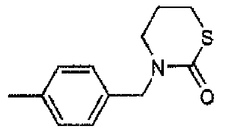 |

**Table 3**

| | | |
|---|---|---|
| 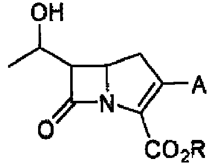 | | |

| Compound No. | R | A |
|---|---|---|
| 17 | -CH₂OCO t-Bu | 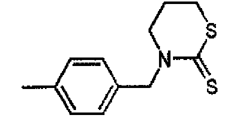 |
| 18 | -CH₂OCO t-Bu | 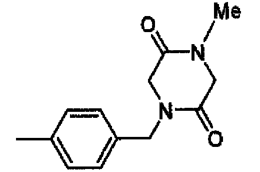 |
| 19 | -CH₂OCO t-Bu | 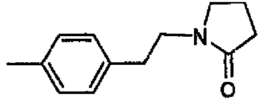 |
| 20 | -CH₂OCO t-Bu | 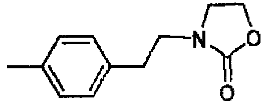 |
| 21 | -CH₂OCO t-Bu | 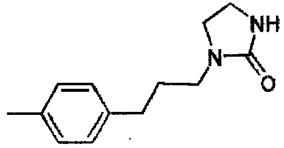 |
| 22 | -CH₂OCO t-Bu | 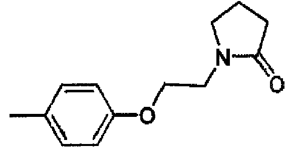 |
| 23 | 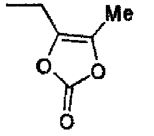 | 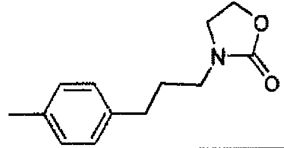 |
| 24 | 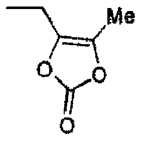 | 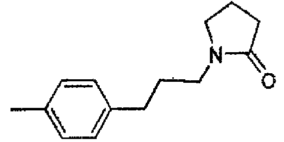 |

**Table 4**

| | | |
|---|---|---|
| 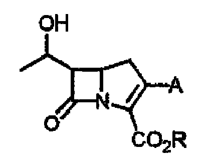 | | |

| Compound No. | R | A |
|---|---|---|
| 25 | H | 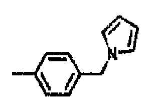 |
| 26 | H | 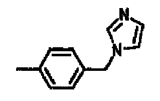 |
| 27 | H | 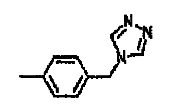 |
| 28 | H | 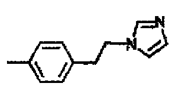 |
| 29 | H | 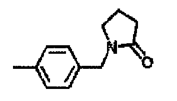 |
| 30 | H | 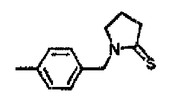 |
| 31 | H | 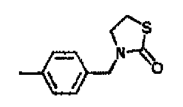 |
| 32 | H | 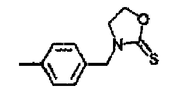 |

**Table 5**

| | | |
|---|---|---|
| 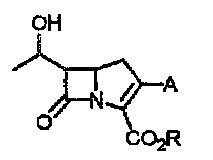 | | |

| Compound No. | R | A |
|---|---|---|
| 33 | H | 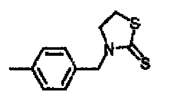 |
| 34 | H | 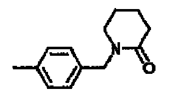 |
| 35 | H | 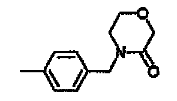 |
| 36 | H | 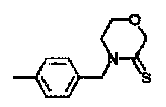 |
| 37 | H | 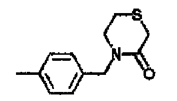 |
| 38 | H | 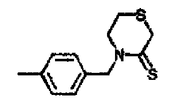 |
| 39 | H | 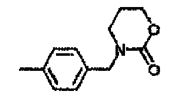 |
| 40 | H | 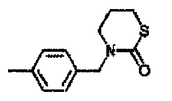 |

**Table 6**

| | | |
|---|---|---|
| 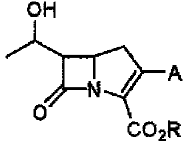 | | |

| Compound No. | R | A |
|---|---|---|
| 41 | H | 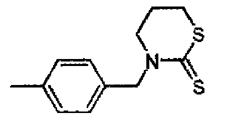 |
| 42 | H | 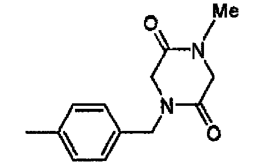 |
| 43 | H | 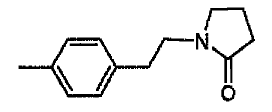 |
| 44 | H | 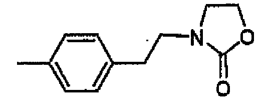 |
| 45 | H | 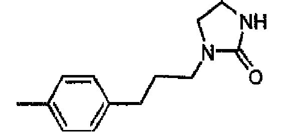 |
| 46 | H | 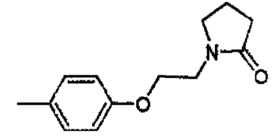 |
| 47 | H | 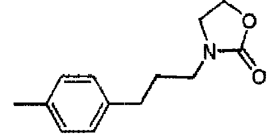 |
| 48 | H | 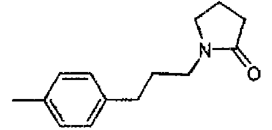 |

### Example

The present invention is explained by illustrating Examples, but is not limited by such Examples.

The abbreviated terms used in Examples mean as follows.
Ac: acetyl group
ALOC: allyloxycarbonyl group
br.: broad
t-Bu: tert-butyi group
DMF: N,N-dimethylformamide
Ph: phenyl group
TBDMS: tert-butyl(dimethyl)silyl group
THF: tetrahydrofuran
TMS: trimethylsilyl group

### Example 1

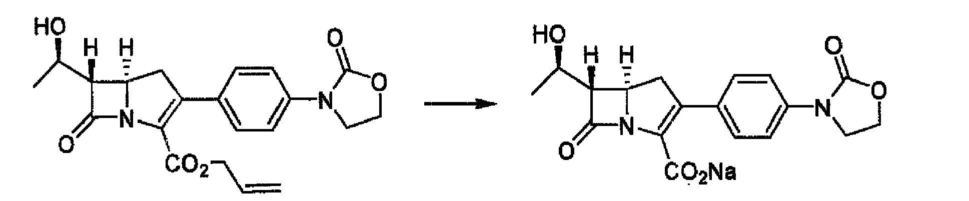

Allyl (5R,6S)-6-[(1R)-1-hydroxyethyl]-7-oxo-3-[4-(2-oxo-1,3-oxazolidin-3-yl)phenyl]-1-azabicyclo[3.2.0]hept-2-ene-2-carboxylate (0.49 g, 1.2 mmol) obtained by reference example 3, and triphenylphosphine (31 mg, 0.12 mmol) were dissolved in THF (30 ml), and to the solution were added at room temperature sodium 2-ethylhexanoate (0.20 g, 1.2 mmol) and tetrakis(triphenylphosphine)palladium(0) (68 mg, 0.06 mmol), followed by stirring for 20 minutes. Thereto was added hexane (20 ml) and resulting white solid was filtered under a nitrogen atmosphere, washed with hexane-THF (1:5) and dried in vacuo at room temperature to give a crude product. The product was dissolved in a small amount of ice water and purified by C18 reverse-phase column chromatography (filler: Wako Pure Chemical; Wakosil 40C18, mobile phase; 0~2%THF/ice-cooled ion-exchange water). The combined fraction containing the object compound was stirred for 1 hour at room temperature in vacuo to remove THF. The residue was lyophilized to give sodium (5R,6S)-6-[(1R)-1-hydroxyethyl]-7-oxo-3-[4-(2-oxo-1,3-oxazolidin-3-yl) phenyl]-1-azabicyclo[3.2.0]hept-2-ene-2-carboxylate (348 mg, yield 78%).
¹H NMR (400 MHz, D₂O) δ 1.07 (d, 3 H, J = 6.4 Hz), 2.85 (dd, 1 H, J = 16.9, 9.8 Hz), 3.21 (dd, 1 H, J = 17.0, 8.5 Hz), 3.28 (dd, 1 H, J = 6.0, 2.8 Hz), 3.93 (dd, 2 H, J = 8.6, 6.7 Hz), 3.95-4.12 (m, 2 H), 4.31 (dd, 2 H, J = 8.6, 6.7 Hz), 7.12-7.23 (m, 4 H).

### Example 2

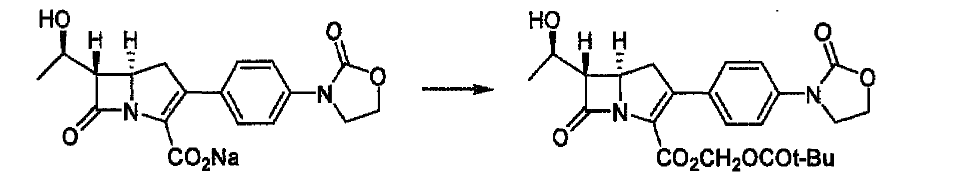

Sodium (5R,6S)-6-[(1R)-1-hydroxyethyl]-7-oxo-3-[4-(2-oxo-1,3-oxazolidin-3-yl)phenyl]-1-azabicyclo[3.2.0]hept-2-ene-2-carboxylate (0.15g, 0.39 mmol) obtained by Example 1 was dissolved in dried DMF (8.0 ml) and therein was gradually dropped under ice cooling pivaloyloxymethyl iodide (105 mg), followed by stirring. One hour later, the reaction mixture was diluted with ethyl acetate, washed with an aqueous sodium hydrogen carbonate solution, water and brine (twice), successively. The organic layer was dried over sodium sulfate and concentrated, and the residue was purified with silica gel column chromatography (silica gel 10 g, hexane: ethyl acetate =1:2 → ethyl acetate only) to give [(2,2-dimethylprapanayl)oxy]methyl (5R,6S)-6-[(1R)-1-hydroxyethyl]-7-oxo-3-[4-(2-oxo-1,3-oxazolidin-3-yl)phenyl]-1-azabicyclo[3.2.0]hept-2-ene-2-carboxylate (0.15g, yield 84%).
¹H NMR (400 MHz, CDCl₃) δ1.19 (s, 9 H), 1.37 (d, 3 H, J = 6.3 Hz), 1.79 (br. s, 1. H), 3.17-3.35 (m, 3 H), 4.08 (t, 2 H, J = 5.2 Hz), 4.16-4.30 (m, 2 H), 4.51 (t, 2 H, J = 5.2 Hz), 5.78 (d, 1 H, J = 5.5 Hz), 5.87 (d, 1: H, J = 5.5 Hz), 7.41 (d, 2 H, 8.6 Hz), 7.55 (d, 2 H, 8.6 Hz).

### Example 3

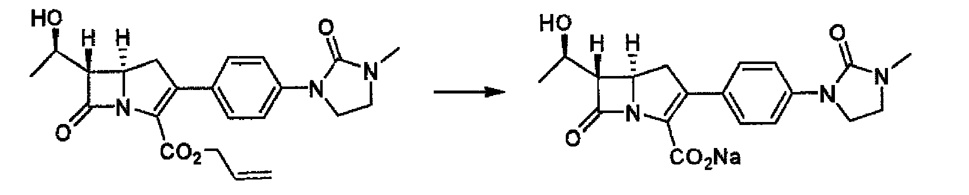

Allyl (5R,6S)-6-[(1R)-1-hydroxyethyl]-3-[4-(3-methyl-2-oxoimidazolodin-1-yl)phenyl]-7-oxo-1-azabicyclo[3.2.0]hept-2-ene-2-carboxylate (0.61 g, 1.0 mmol) obtained by reference example 6 and dichloro bis(triphenylphosphine)palladium(II) (18 mg, 0.025 mmol) were dissolved in methylene chloride (40 ml), and thereto was added at 0°C tri-n-butyltin hydride (4.4 g, 15 mmol), followed by stirring for 30 minutes. To the reaction mixture was dropped an aqueous sodium hydrogen carbonate solution (0.20M, 10ml), and the aqueous layer was washed with diethyl ether and separated by a separating funnel. The aqueous layer was concentrated at 0°C and the residue was purified by C 18 reverse-phase column chromatography (filler: Wako Pure Chemical; Wakosil 40C18, mobile phase; 0~2%THF/ice-cooled ion-exchange water). The combined fraction containing the object compound was stirred in vacuo for 1 hour at room temperature to remove THF, and lyophilized to give sodium (5R,6S)-6-[(1R)-1-hydroxyethyl]-3-[4-(3-methyl-2-oxoimidazolidin-1-yl)phenyl]-7-oxo-1-azabicyclo[3.2.0]hepto-2-ene-2-carboxylate (283 mg, yield 72%).
¹H NMR (400 MHz, D₂O) δ 1.13 (d, 3 H, J = 6.4 Hz), 2.67 (s, 3 H), 2.89 (dd, 1 H, J = 16.9, 9.8 Hz), 3.24 (dd, 1 H, J = 17.0, 8.5 Hz), 3.31 (dd, 1 H, J = 6.0, 2.7 Hz), 3.32-3.39 (m, 2 H), 3.65-3.74 (m, 2 H), 4.02-4.17 (m, 2 H), 7.15-7.24 (m, 4 H).

### Example 4

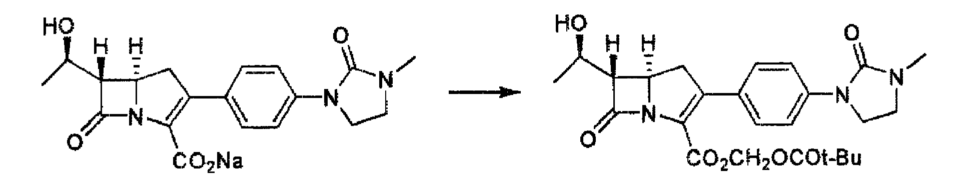

Sodium (5R,6S)-6-[(1R)-1-hydroxyethyl]-3-[4-(3-methyl-2-oxoimidazolidin-1-yl)phenyl]-7-oxo-1-azabicycio[3.2.0]hegt-2-ene-2-carboxylate (0.11g) obtained by example 3 was dissolved in dried DMF (6 ml), and thereto was gradually dropped under ice cooling pivaloyloxymethyl iodide (72 mg), followed by stirring. One hour later, the reaction mixture was diluted with ethyl acetate and washed with an aqueous sodium hydrogen carbonate solution, water and brine, successively. The organic layer was dried over anhydrous sodium sulfate and concentrated, and the residue was purified by silica gel column chromatography (silica gel 10g, hexane : ethyl acetate = 1:1 → ethyl acetate only) to give [(2,2-dimethylpropanoyl)oxy]methyl (5R,6S)-6-[(1R)-1-hydroxyethyl]-3-[4-(3-methyl-2-oxoimidazolidin-1-yl)phenyl]-7-oxo-1-azabicyclo[3.2.0]hept-2-ene-2-carboxylate (0.10g, yield 78%).
¹H NMR (400 MHz, CDCl₃) δ1.19 (s, 9 H), 1.37 (d, 3 H, J = 6.3 Hz), 1.79 (br. s, 1 H), 2.91 (s, 3 H), 3.18-3.36 (m, 3 H), 3.49 (dd, 2 H, J = 8.2, 6.0 Hz), 3.81 (dd, 2 H, J = 9.9, 7.7 Hz), 4.20-4.32 (m, 2 H), 5.80 (d, 1 H, J = 5.5 Hz), 5.88 (d, 1 H, J = 5.5 Hz), 7.33-7.39 (m, 2 H), 7.52-7.59 (m, 2 H) .

### Example 5

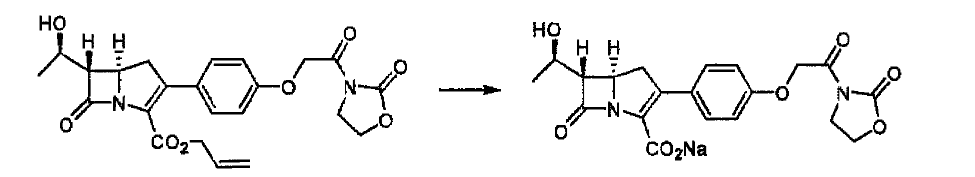

Allyl (5R,6S)-6-[(1R)-1-hydroxyethyl]-7-oxo-3-{4-[2-oxo-2-(2-oxo-1,3-oxazolidin-3-yl)ethoxy]phenyl}-1-azabicyclo[3.2.0]hept-2-ene-2-carboxylate (0.36 g, 0.80 mmol) obtained by reference example 8 and triphenylphosphine (21 mg, 0.080 mmol) were dissolved in THF (25 ml), and thereto were added at 0°C sodium 2-ethylhexanoate (133 mg, 0.80 mmol), tetrakis(triphenylphosphine)palladium(0) (46 mg, 0.040 mmol) and 2-propanol (10 ml), followed by stirring for 30 minutes. Thereto was added hexane (20 ml) and resulting white solid was filtered under a nitrogen atmosphere, washed with hexane/THF (1:5) and dried in vacuo at room temperature to give a crude product. The product was dissolved in a small amount of ice water and purified by C 18 reverse-phase column chromatography (filler: Wako Pure Chemical; Wakosil 40C18, mobile phase; 0~3%THF/ice-cooled ion-exchange water). The combined fraction containing the object compound was stirred in vacuo for 1 hour at room temperature to remove THF. The residue was lyophilized to give sodium (5R,6S)-6-[(1R)-1-hydroxyethyl]-7-oxo-3-{4-[2-oxo-2-(2-oxo-1,3-oxazolidin-3-yl)ethoxy]phenyl}-1-azabicyclo[3.2.0]hept-2-ene-2-carboxylate (224 mg, yield 66%).
¹H NMR (400 MHz, D₂O) δ 1.12 (d, 3 H, J = 6.0 Hz), 2.88 (dd, 1 H, J = 17.0, 9.8 Hz), 3.22 (dd, 1 H, J = 17.0, 8.6 Hz), 3.29 (dd, 1 H, 6.0, 2.8 Hz), 3.88 (t, 2 H, J = 8.4 Hz), 4.01-4.14 (m, 2 H), 4.44 (t, 2 H, J = 8.4 Hz), 5.15 (s, 2 H), 6.74-6.83 (m, 2 H), 7.12-7.I9 (m, 2 H).

### Example 6

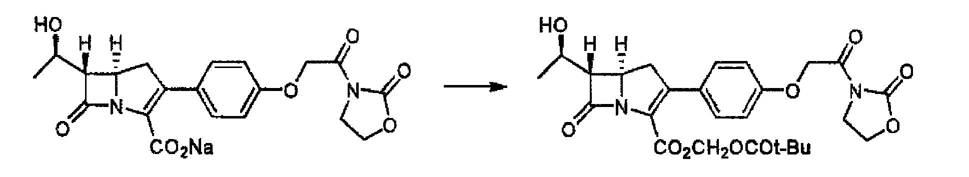

Sodium (5R,6S)-6-[(1R)-1-hydroxyethyl]-7-oxo-3-{4-[2-oxo-2-(2-oxo-1,3-oxazolidin-3-yl)ethoxy]phenyl]-1-azabicyclo[3.2.0]hept-2-ene-2-carboxylate (91 mg) obtained by example 5 was dissolved in dried DMF (6 ml), and thereto was gradually dropped under ice cooling pivaloyloxymethyl iodide (57 mg), followed by stirring. One hour later, the reaction mixture was diluted with ethyl acetate, washed with an aqueous sodium hydrogen carbonate solution, water and brine (twice), successively. The organic layer was dried over anhydrous sodium sulfate and concentrated, and the residue was purified by silica gel column chromatography (silica gel 10 g, hexane : ethyl acetate = 1:1 → 1:7) to give [(2,2-dimethylpropanoyl)oxy]methyl (5R,6S)-6-[(1R)-1-hydroxyethyl]-7-oxo-3-{4-[2-oxo-2-(2-oxo-1,3-oxazolidin-3-yl)ethoxy]phenyl}-1-azabicyclo[3.2.0]hepto-2-ene-2-carboxylate (60 mg, yield 54%).
¹H NMR (400 MHz, CDCl₃) δ1.15 (s, 9 H), 1.37 (d, 3 H, J = 6.3 Hz), 1.73 (d, 1 H, J = 4.8 Hz), 3.15-3.33 (m, 3 H), 4.04-4.12 (m, 2 H), 4.20-4.30 (m, 2 H), 4.51-4.58 (m, 2 H), 5.25 (s, 2 H), 5.78 (d, 1 H, J = 5.5 Hz), 5.87 (d, 1 H, J = 5.5 Hz), 6.85-6.92 (m, 2 H), 7.33-7.38 (m, 2 H).

### Example 7

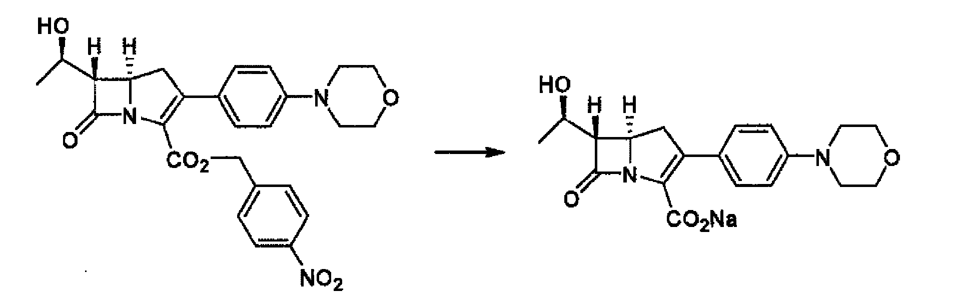

To a solution of 4-nitrobenzyl (5R,6S)-6-[(1R)-1-hydroxyethyl]-3-(4-morpholin-4-ylphenyl)-7-oxo-1-azabicyclo[3.2.0]hepto-2-ene-2-carboxylate (0.65 g, 1.3 mmol) in THF (20 ml) obtained by reference example 9 were added 10%palladium/carbon (65 mg) and sodium hydrogen carbonate (0.22 g, 2.7 mmol), and the mixture was stirred for 1 hour at 0°C under a hydrogen atmosphere (ordinary pressure). The reaction mixture was filtrated over Celite and washed with THF-water (1:1) (15 ml). The aqueous layer was washed with diethyl ether and separated by a separating funnel. The aqueous layer was concentrated at 0°C, and the residue was purified by C18 reverse-phase column chromatography (filler: Wako Pure Chemical; Wakosil 40C18, mobile phase; 0~2%THF/ice-cooled ion-exchange water). The combined fraction containing the object compound was stirred in vacuo at room temperature to remove THF and lyophilized to give sodium (5R,6S)-6-[(1R)-1-hydroxyethyl]-3-(4-morphlin-4-ylphenyl)-7-oxo-1-azabicyclo[3.2.0]hepto-2-ene-2-carboxylate (205 mg, yield 41%).
¹H NMR (400 MHz, D₂O) δ 1.13 (d, 3 H, J = 6.4 Hz), 2.88 (dd, 1 H, J = 16.9, 9.8 Hz), 2.94-3.06 (m, 4 H), 3.23 (dd, 1H, J = 17.0, 8.6 Hz), 3.30 (dd, 1 H, J = 6.0, 2.7 Hz), 3.65-3.78 (m, 4 H), 4.02-4.15 (m, 2 H), 6.83-6.91 (m, 2 H), 7.12-7.21 (m, 2 H).

### Example 8

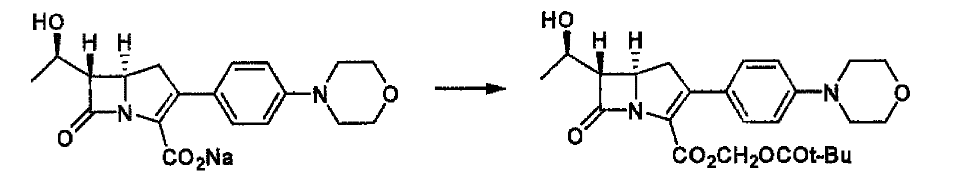

Sodium (5R,6S)-6-[(1R)-1-hydroxyethyl]-3-(4-morphlin-4-ylphenyl)-7-oxo-1-azabicyclo[3.2.0]hepto-2-ene-2-carboxylate (94 mg) obtained by example 7 was dissolved in dried DMF (4 ml), and thereto was gradually dropped under ice cooling pivaloyloxymethyl iodide (66 mg), followed by stirring. One hour later, the reaction mixture was diluted with ethyl acetate, washed with an aqueous sodium hydrogen carbonate solution, water and brine (twice), successively. The organic layer was dried over anhydrous sodium sulfate and concentrated, and the residue was purified by silica gel column chromatography (silica gel 15 g, hexane : ethyl acetate = 1:1 → 1:3) to give [(2,2-dimethylpropanoyl)oxy]methyl (5R,6S)-6-[(1R)-1-hydroxyethyl]-3-(4-morpholin-4-ylphenyl)-7-oxo-1-azabicyclo[3.2.0]hepto-2-ene-2-carboxylate (96 mg, yield 82%).
¹H NMR (400 MHz, CDCl₃) δ1.20 (s, 9 H), 1.36 (d, 3 H, J = 6.3 Hz), 1.75 (d, 1 H, 4.8 Hz), 3.14-3.33 (m, 7 H), 3.80-3.88 (m, 4 H), 4.21-4.33 (m, 2 H), 5.80 (d, 1 H, J = 5.5 Hz), 5.89 (d, 1 H, J = 5.5 Hz), 6.84 (d, 2 H, J = 9.0 Hz), 7.38 (d, 2 H, J = 9.0 Hz), 7.82.

### Example 9

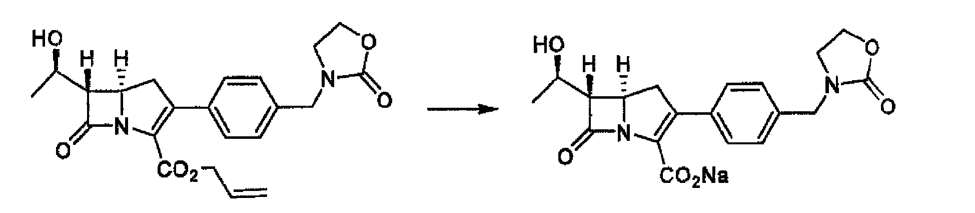

Allyl (5R,6S)-6-[(1R)-1-hydroxyethyl]-7-oxo-3-{4-[(2-oxo-1,3-oxazolidin-3-yl)methyl]phenyl}-1-azabicyclo[3.2.0]hepto-2-ene-2-carboxylate (0.58 g, 1.4 mmol) obtained by reference example 11 and dichloro bis(triphenylphosphine)palladium(II) (25 mg, 0.035 mmol) were dissolved in methylene chloride (25 ml), and thereto was added at 0°C tri-n-butyltin hydride (6.2 g, 21 mmol), followed by stirring for 20 minutes. To the reaction mixture was dropped an aqueous sodium carbonate solution (0.14M, 10ml) and the aqueous layer was washed with diethyl ether and separated by a separating funnel. The aqueous layer was concentrated at 0°C and the residue was purified by C18 reverse-phase column chromatography (filler: Wako Pure Chemical; Wakosil 40C18, mobile phase; 0-2%THF/ice-cooled ion-exchange water). The combined fraction containing the object compound was stirred in vacuo at room temperature for 1 hour to remove THF and lyophilized to give sodium (5R,6S)-6-[(1R)-1-hydroxyethyl]-7-oxo-3-{4-[(2-oxo-1,3-oxazolidin-3-yl)methyl]phenyl}-1-azabicyclo[3.2.0]hepto-2-ene-2-carboxylate (372 mg, yield 67%).
¹H NMR (400 MHz, D₂O) δ 1.14 (d, 3 H, J = 6.4 Hz), 2.91 (dd, 1 H, J = 17.0, 9.8 Hz), 3.27 (dd, 1 H, J = 17.0, 8.5 Hz), 3.34 (dd, 1 H, J = 6.0, 2.8 Hz), 3.41 (dd, 2 H, J = 8.4, 7.0 Hz), 4.03-4.18 (m, 2 H), 4.21-4.30 (m, 4 H), 7.14 (d, 2 H, J = 8.3 Hz), 7.21 (d, 2 H, J = 8.3 Hz).

### Example 10

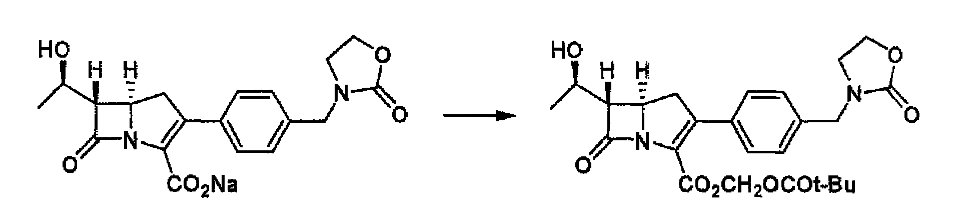

Sodium (5R,6S)-6-[(1R)-1-hydroxyethyl]-7-oxo-3-{4-[(2-oxo-1,3-oxazolidin-3-yl)methyl]phenyl}-1-azabicyclo[3.2.0]hepto-2-ene-2-carboxylate (0.20 g) obtained by example 9 was dissolved in dried DMF (15 ml). Thereto was gradually dropped under ice-cooling pivaloyloxymethyl iodide (0.16 g), followed by stirring. One hour later, the reaction mixture was diluted with ethyl acetate, washed with an aqueous sodium hydrogen carbonate solution, water and brine (three times), successively. The organic layer was dried over anhydrous sodium sulfate and concentrated, and the residue was purified by silica gel column chromatography (silica gel 10 g, hexane : ethyl acetate = 1:1 → ethyl acetate only) to give [(2,2-dimethylpropanoyl)oxy]methyl (5R,6S)-6-[(1R)-1-hydroxyethyl]-7-oxo-3-{4-[(2-oxo-1,3-oxazolidin-3-yl)methyl]phenyl}-1-azabicyclo[3.2.0]hepto-2-ene-2-carboxylate (0.20 g, yield 74%).
¹H NMR (400 MHz, CDCl₃) δ1.20 (s, 9 H), 1.37 (d, 3 H, J = 6.3 Hz), 1.71 (br. s, 1 H), 3.18-3.39 (m, 3 H), 3.46 (t, 2 H, J = 8.2 Hz), 4.22-4.36 (m, 4 H), 4.44 (s, 2 H), 5.77 (d, 1 H, J = 5.5 FIz), 5.86 (d, 1 H, J = 5.5 Hz), 7.23-7.39 (m, 4 H).

### Example 11

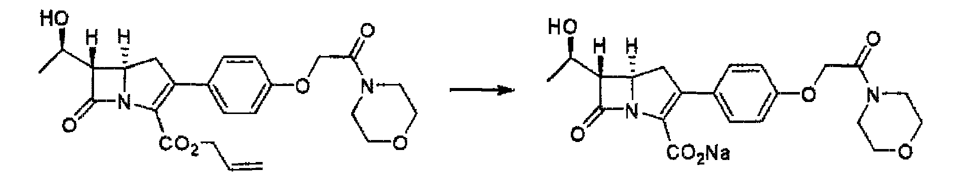

Allyl (5R,6S)-6-[(1R)-1-hydroxyethyl]-3-[4-(2-morpholin-4-yl-2-oxoethoxy)phenyl]-7-oxo-1-azabicyclo[3.2.0]hept-2-ene-2-carboxylate (0.59 g, 1.3 mmol) obtained by reference example 13 and dichloro bis(triphenylphosphine)palladium(II) (23 mg, 0.032 mmol) were dissolved in methylene chloride (50 ml), and thereto was added at 0°C tri-n-butyltin hydride (5.8 g, 19 mmol), followed by stirring for 20 minutes. To the reaction mixture was dropped an aqueous sodium hydrogen carbonate solution (0.26 M, 10 ml), and the aqueous layer was washed with diethyl ether and separated by a separating funnel. The aqueous layer was concentrated at 0°C and the residue was purified by C18 reverse-phase column chromatography (filler: Wako Pure Chemical; Wakosil 40C 18, mobile phase; 0~2%THF/ice-cooled ion-exchange water). The combined fraction containing the object compound was stirred in vacuo for 1 hour at room temperature to remove THF. The residue was lyophilized to give sodium (5R,6S)-6-[(1R)-1-hydroxyethyl]-3-[4-(2-morpholin-4-yl-2-oxoethoxy)phenyl]-7-oxo-1-azabicyclo[3.2.0]hepto-2-ene-2-carboxylate (0.35 g, yield 64%).
¹H NMR (400 MHz, D₂O) δ 1.13 (d, 3 H, J = 6.4 Hz), 2.89 (dd, 1 H, J = 17.0, 9.8 Hz), 3.24 (dd, 1 H, J = 17.0, 8.5 Hz), 3.42-3.49 (m, 4 H), 3.56-3.65 (m, 4 H), 4.03-4.16 (m, 2 H), 4.80 (s, 2 H), 6.74-6.80 (m, 2 H), 7.12-7.19 (m, 2 H).

### Example 12

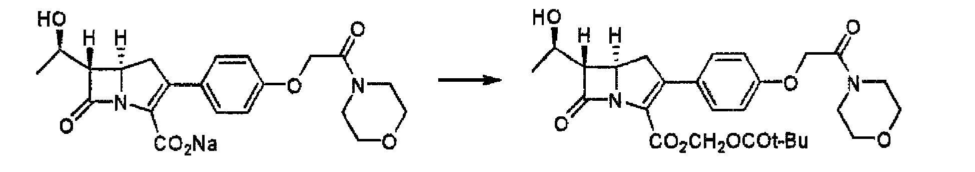

Sodium (5R,6S)-6-[(1R)-1-hydroxyethyl]-3-[4-(2-morpholin-4-yl-2-oxoethoxy)phenyl]-7-oxo-1-azabicyclo[3.2.0]hept-2-ene-2-carboxylate (0.16 g) obtained by example 11 was dissolved in dried DMF (8 ml), and thereto was gradually dropped under ice cooling pivaloyloxymethyl iodide (94 mg), followed by stirring. One hour later, the reaction mixture was diluted with ethyl acetate, washed with an aqueous sodium hydrogen carbonate solution, water and brine (three times), successively. The organic layer was dried over anhydrous sodium sulfate and concentrated, and the residue was purified by silica gel column chromatography (silica gel 20 g, hexane : ethyl acetate = 1:1 → ethyl acetate only) to give [(2,2-dimethylpropanol)oxy]methyl (5R,6S)-6-[(1R)-1-hydroxyethyl]-3-[4-(2-morpholin-4-yl-2-oxoethoxy)phenyl]-7-oxo-1-azabicyclo[3.2.0]hept-2-ene-2-carboxylate (0.15 g, yield 82%).
¹H NMR (400 MHz, CDCl₃) δ1.20 (s, 9 H), 1.37 (d, 3 H, J = 6.3 Hz), 1.80 (br. s, 1 H), 3.13-3.31 (m, 3 H), 3.54-3.70 (m, 8 H), 4.21-4.29 (m, 2 H), 4.70 (s, 2 H), 5.78 (d, 1 H, J = 5.5 Hz), 5.87 (d, 1 H, J = 5.5 Hz), 6.87-6.91 (m, 2 H), 7.32-7.37 (m, 2 H).

### Example 13

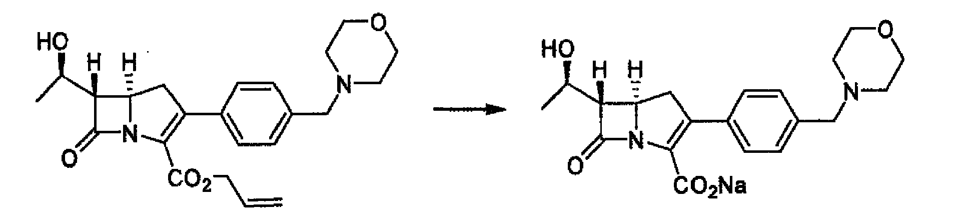

Allyl (5R,6S)-6-[(1R)-1-hydroxyethyl]-3-[4-(2-morpholin-4-yl-2-oxoethoxy)phenyl]-7-oxo-1-azabicyclo[3.2.0]hept-2-ene-2-carboxylate (0.41 g, 1.0 mmol) obtained by reference example 15 and dichloro bis(triphenylphosphine)palladium(II) (18 mg, 0.027 mmol) were dissolved in methylene chloride (20 ml), and thereto was added at 0°C tri-n-butyltin hydride (4.4 g, 15 mmol), followed by stirring for 20 minutes. To the reaction mixture was dropped an aqueous sodium hydrogen carbonate solution (0.20 M, 5 ml) and the aqueous layer was washed with diethyl ether and separated by a separating funnel. The aqueous layer was concentrated at 0°C and the residue was purified by C18 reverse-phase column chromatography (filler: Wako Pure Chemical; Wakosil 40C 18, mobile phase; 0∼3% THF/ice-cooled ion-exchange water). The combined fraction containing the object compound was stirred in vacuo for 1 hour at room temperature to remove THF. The residue was lyophilized to give sodium (5R,6S)-6-[(1R)-1-hydroxyethyl]-3-[4-(morpholin-4-ylmethyl)phenyl]-7-oxo-1-azabicyclo[3.2.0]hept-2-ene-2-carboxylate (0.28 g, yield 70%).
¹H NMR (400 MHz, D₂O) δ 1.13 (d, 3 H, J = 6.4 Hz), 2.42 (br. s, 4 H), 2.90 (dd, 1 H, J = 17.0, 9.8 Hz), 3.24 (dd, 1 H, J = 17.0, 8.5 Hz), 3.33 (dd, 1 H, J = 6.0, 2.8 Hz), 3.43 (s, 2 H), 3.56 (br. s, 4 H), 4.03-4.17 (m, 2 H), 7.10-7.22 (m, 4 H).

### Example 14

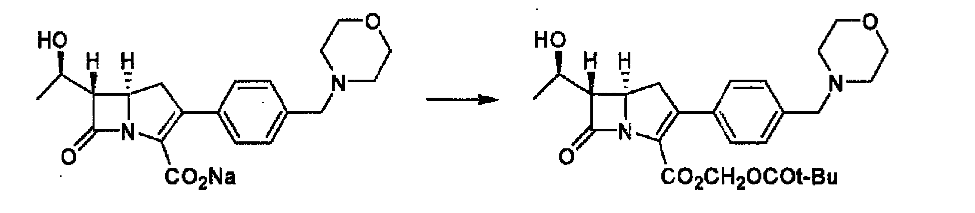

Sodium (5R,6S)-6-[(1R)-1-hydroxyethyl]-3-[4-(morpholin-4-ylmethyl)phenyl]-7-oxo-1-azabicyclo[3.2.0]hept-2-ene-2-carboxylate (0.14 g) obtained by example 13 was dissolved in dried DMF (6 ml), and thereto were added benzyltriethylammonium chloride (0.24 g) and pivaloyloxymethyl chloride (0.16 g) under ice cooling, followed by stirring for 14 hours. To the reaction mixture was added the ethyl acetate, and the mixture was washed with an aqueous sodium hydrogen carbonate solution, water and brine (three times), successively. The organic layer was dried over anhydrous sodium sulfate and concentrated, and the residue was purified by silica gel column chromatography (silica gel 10 g, hexane : ethyl acetate = 1:1 → ethyl acetate only) to give [(2,2-dimethylpropanoyl)oxy]methyl (5R,6S)-6-[(1R)-1-hydroxyethyl]-3-[4-(morpholin-4-ylmethyl)phenyl]-7-oxo-1-azabicyclo[3.2.0]hept-2-ene-2-carboxylate (0.13 g, yield 74%).
¹H NMR (400 MHz, CDCl₃) δ1.20 (s, 9 H), 1.36 (d, 3 H, J = 6.3 Hz), 1.89 (br. s, 1 H), 2.47 (br. s, 4 H), 3.14-3.35 (m, 3 H), 3.50 (s, 2 H), 3.73 (s, 4 H), 4.19-4.31 (m, 2 H), 5.76 (d, 1 H, J = 5.5 Hz), 5.87 (d, 1 H, J = 5.5 Hz), 7.30-7.37 (m, 4 H).

### Example 15

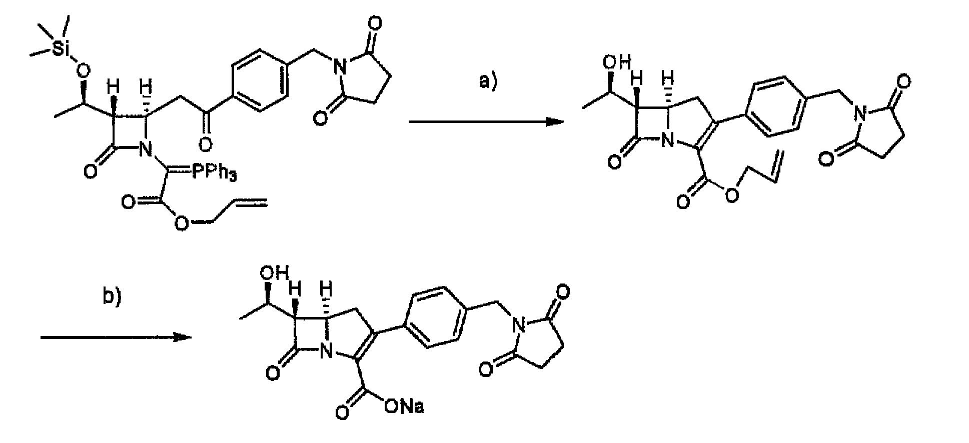

### Step a)

Allyl (2R,3S)-2-(2-{4-[(2,5-dioxopyrrolidin-1-yl)methyl]phenyl}-2-oxoethyl)-4-oxo-3-{(1R)-1-[(trimethylsilyl)oxy]ethyl}azetidin-1-yl)(triphenylphosphoranilidene) acetate (1.58g, 2.03mmol) obtained by reference example 16 was dissolved in toluene (32 ml), and thereto were added N,O-bistrimethylsilylacetamide (1.0 ml, 4.06 mmol) and hydroquinone (20 mg), followed by refluxing under heating at 100°C for 7 hours. After being cooled, the reaction mixture was concentrated, and the residue was purified by silica gel column chromatography (silica gel 120 ml, chloroform: methanol = 100:0 ~ 100:3) to give a yellow oil (2.06 g). This product without further purification was dissolved in THF (40 ml) and water (20 ml) and thereto was gradually dropped 0.1 N hydrochloric acid under cooling in an ice bath with a pH meter to adjust pH to 3.0. After 6 minutes, thereto was gradually dropped a saturated aqueous sodium hydrogen carbonate solution to adjust pH to 6.8, and saturated brine (50 ml) was added thereto. After extracting with ethyl acetate, the combined organic layer was dried over anhydrous sodium sulfate, filtered, and concentrated to give allyl (5R,6S)-3-{4-[(2,5-dioxopyrrolidin-1-yl)methyl]phenyl}-6-[(1R)-1-hydroxyethyl]-7-oxo-1-azabicyclo[3.2.0]hepto-2-ene-2-carboxylate (0.76 g, yield 88%) as a yellow amorphous. The product was used for next reaction without further purification.
¹H NMR (400 MHz, CDCl₃) δ 1.37 (d, 3 H, J = 6.0 Hz), 1.84 (br. s, 1 H), 2.71 (s, 4 H), 3.13-3.32 (m, 3 H), 4.22-4.33 (m, 2 H), 4.58-4.76 (m, 2 H), 4.84 (s, 2 H), 5.17-5.29 (m, 2 H), 5.81-5.92 (m, 1 H), 7.28-7.34 (m, 2 H), 7.36-7.42 (m, 2 H).

### Step b)

Allyl (5R,6S)-3-{4-[(2,5-dioxopyrrolidin-1-yl)methyl]phenyl}-6-[(1R)-1-hydroxyethyl]-7-oxo-1-azabicyclo[3.2.0]hepto-2-ene-2-carboxylate (0.76 g, 1.80 mmol) obtained by the above step was dissolved in THF (20 ml) under ice cooling, and thereto were added triphenylphosphine (47.2mg, 0.18mmol), sodium 2-ethylhexanoate (389 mg, 2.3 mmol), and tetrakis triphenylphosphinepalladium (104 mg, 0.09 mmol), successively. After stirring for 15 minutes, thereto was gradually dropped cooled hexane (10 ml) to prepare a suspension of the reaction mixture. After stirring additional 20 minutes, the suspension was filtered under a nitrogen atmosphere. The filtered solid was washed with cooled hexane/cooled THF (volume ratio = 1/1) (60 ml), dried in vacuo to give crude sodium (5R,6S)-3-{4-[(2,5-dioxopyrrolidin-1-yl)methyl]phenyl}-6-[(1R)-1-7-oxo-1-azabicyclo[3.2.0]hepta-2-ene-2-carboxylate. This product was suspended in ice-cooled ion-exchange water (20ml) and purified by C 18 reverse-phase column chromatography (Wakosil C18 reverse-phase column, mobile phase; cooled ion-exchange water/THF = 100:0∼100:2) and then lyophilized to give sodium (5R,6S)-3-{4-[(2,5-dioxopyrrolidin-1-yl)methyl]phenyl}-6-[(1R)-1-7-oxo-1-azabicyclo[3.2.0]hepto-2-ene-2-carboxylate (380 mg, yield 52%).
¹H NMR (400 MHz, D₂O) δ1.20 (d, 3H, J=6.4Hz), 2.70 (s,4H), 2.94 (dd, 1H, J=17.2Hz, 8.8Hz), 3.29 (dd, 1H, J=17.2Hz, 8.4Hz), 3.37.-3.40 (m, 1 4.11-4.20 (m,2H), 4.55 (s,1H), 7.16 (d,2H, J=8.4Hz), 7.26 (d,2H, J=8.8Hz).

### Example 16

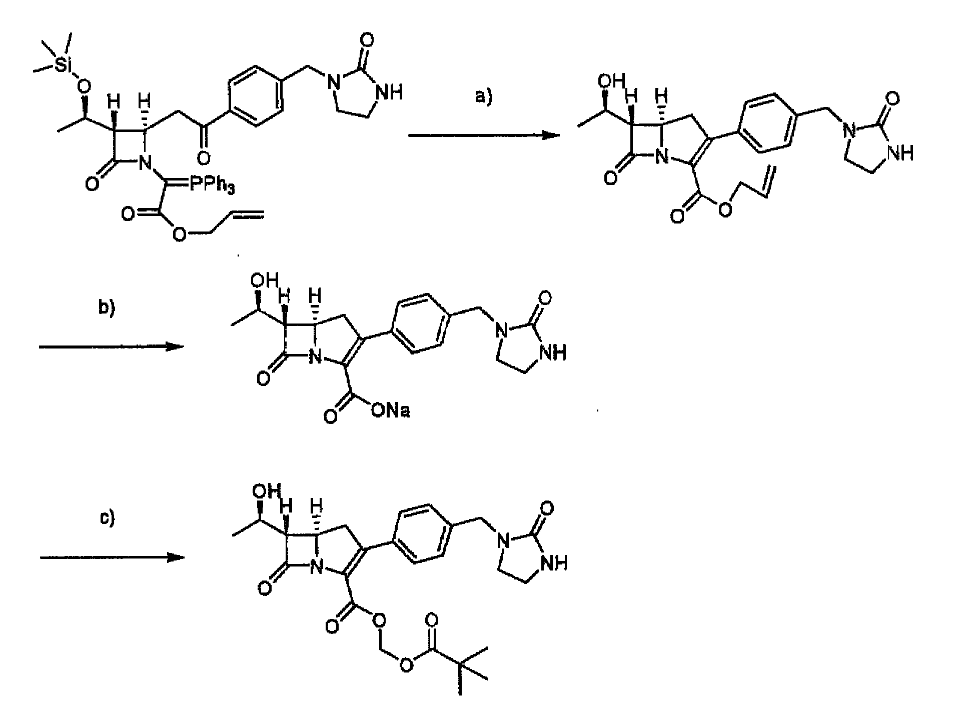

### Step a)

Allyl (3S,4R)-2-oxo-4-[2-oxo-2-(4-{[2-oxo-3-(trimethylsilyl)imidazolidin-1-yl]methyl}phenyl)ethyl]-3-{(1R)-1-[(trimethylsilyl)oxy]ethyl}azetidin-1-yl)(triphenylphosphoranilidene) acetate (0.76 g, 0.99 mmol) obtained by reference example 17 was dissolved in toluene (20 ml), and thereto were added N,O-bistrimethylsilylacetamide (0.49 ml, 2.0 mmol) and hydroquinone (20 mg), followed by refluxing under heating at 100°C for 30 minutes. Thereto was added m-xylene (20 ml) and the mixture was refluxed at 140°C. Two hours later, the reaction mixture was allowed to cool, concentrated, and the residue was purified by silica gel column chromatography (silica gel 100 ml, hexane : ethyl acetate = 4:1~1:2) to give a pale yellow oil (0.37 g). This product without further purification was dissolved in THF (12 ml) and water (6ml), and thereto was gradually dropped 0.1 N hydrochloric acid under cooling in an ice bath with a pH meter to adjust pH to 3.0. After 6 minutes, thereto was gradually dropped a saturated aqueous sodium hydrogen carbonate solution to adjust pH to 6.8, and saturated brine (50 ml) was added thereto. After extracting with ethyl acetate, the combined organic layer was dried over anhydrous sodium sulfate, filtered, and concentrated to give (5R,6S)-6-[(1R)-1-hydroxyethyl]-7-oxo-3-{4-[(2-oxoimidazolidin-1-yl)methyl]phenyl}-1-azabicyclo[3.2.0]hepto-2-ene-2-carboxylate (0.27 g, yield 69%) as a white amorphous. This product was used for next reaction without further purification.
¹H NMR (400 MHz, CDCl₃) δ 1.38 (d, 3 H, J = 6.4 Hz), 1.86 (br. s, 1 H), 3.15-3.43 (m, 7 H), 4.24-4.35 (m, 2 H), 4.36 (s, 3 H), 4.60-4.68 (m, 1 H), 4.69-4.76 (m, 1 H), 5.17-5.20 (m, 1 H), 5.25-5.30 (m, 1 H), 5.82-5.93 (m, 1 H), 7.26 (d, 2 H, J = 10.4 Hz), 7.32 (d, 2 H, J = 10.4 Hz).

### Step b)

(5R,6S)-6-[(1R)-1-Hydroxyethyl]-7-oxo-3-{4-[(2-oxoimidazolidin-1-yl)methyl]phenyl}-1-azabicyclo[3.2.0]hepto-2-ene-2-carboxylate (0.27 g, 0.656 mmol) obtained by the above step was dissolved in THF (5.4 ml) under ice cooling, and thereto were added triphenylphosphine (17.2mg, 0.06 mmol), sodium 2-ethylhexanoate (142 mg, 0.85 mmol), and tetrakis triphenylphosphine palladium (38mg, 0.033mmol), successively. After stirring for an hour, thereto was gradually dropped cooled hexane (7.4 ml) to prepare a suspension of the reaction mixture. After stirring additional 20 minutes, the suspension was filtered under a nitrogen atmosphere. The filtered solid was washed with cooled hexane / cooled THF (volume ratio = 2/1) (50ml), and dried in vacuo to give crude sodium (5R,6S)-6-[(1R)-1-hydroxyethyl]-7-oxo-3-{4-[(2-oxoimidazolidin-1-yl)methyl]phenyl}-1-azabicyclo[3.2.0]hepto-2-ene-2-carboxylate (250 mg). Part of this product (150 mg) was suspended in ice-cooled ion-exchange water (5 ml) and purified by C18 reverse-phase column chromatography (Wakosil C18 reverse-phase column, mobile phase; cooled ion-exchange water/THF = 100:0~100:3) and then lyophilized to give sodium (5R,6S)-6-[(1R)-1-hydroxyethyl]-7-oxo-3-{4-[(2-oxoimidazolidin-1-yl)methyl]phenyl}-1-azabicyclo[3.2.0]hepta-2-ene-2-carboxylate (47.3 mg, yield 30%).
¹H NMR (400 MHz, D₂O) δ 1.21 (d, 3H, J=6.0Hz), 2.98 (dd, 1H, J=16.8Hz, 10.0Hz), 3.32-3.42 (m, 6H), 4.12-4.27 (m, 4H), 7.18 (d, 2H, J=8.0Hz), 7.25 (d, 2H, J=8.4Hz).

### Step c)

Crude sodium (5R,6S)-6-[(1R)-1-hydroxyethyl]-7-oxo-3-{4-[(2-oxoimidazolidn-1-yl)methyl]phenyl}-1-azabicyclo[3.2.0]hepto-2-ene-2-carboxylate (73 mg, 0.186 mmol) obtained by above step b) was dissolved in dried DMF (4 ml), and the solution was ice-cooled. Thereto were added benzyltriethylammonium chloride (0.102 g) and pivaloyloxymethyl chloride (0.062 g), followed by stirring for 23 hours. To the reaction mixture was added the ethyl acetate, and the mixture was washed with an aqueous sodium hydrogen carbonate solution, water and brine (three times), successively. The organic layer was dried over anhydrous sodium sulfate and concentrated, and the residue was purified by silica gel column chromatography (silica gel 10 g, hexane : ethyl acetate = 1:2 → ethyl acetate only) to give [(2,2-dimethyl)oxy]methyl (5R,6S)-6-[(1R)-1-hydroxyethyl]-7-oxo-3-{4-[(2-oxoimidazolidin-1-yl)methyl]phenyl}-1-azabicyclo[3.2.0]hepto-2-ene-2-carboxylate (38 mg, yield 42%).
¹H NMR (400 MHz, CDCl₃) δ 1.20 (s, 9H), 1.37 (d, 3H, J=6.3Hz), 3.12-3.42 (m, 7H), 4.21-4.33 (m, 2H), 4.37 (s, 3H), 5.77 (d, 1H, J=5.5Hz), 5.86 (d, 1H, J=5.5Hz), 7.18-7.34 (d, 4H).

### Example 17

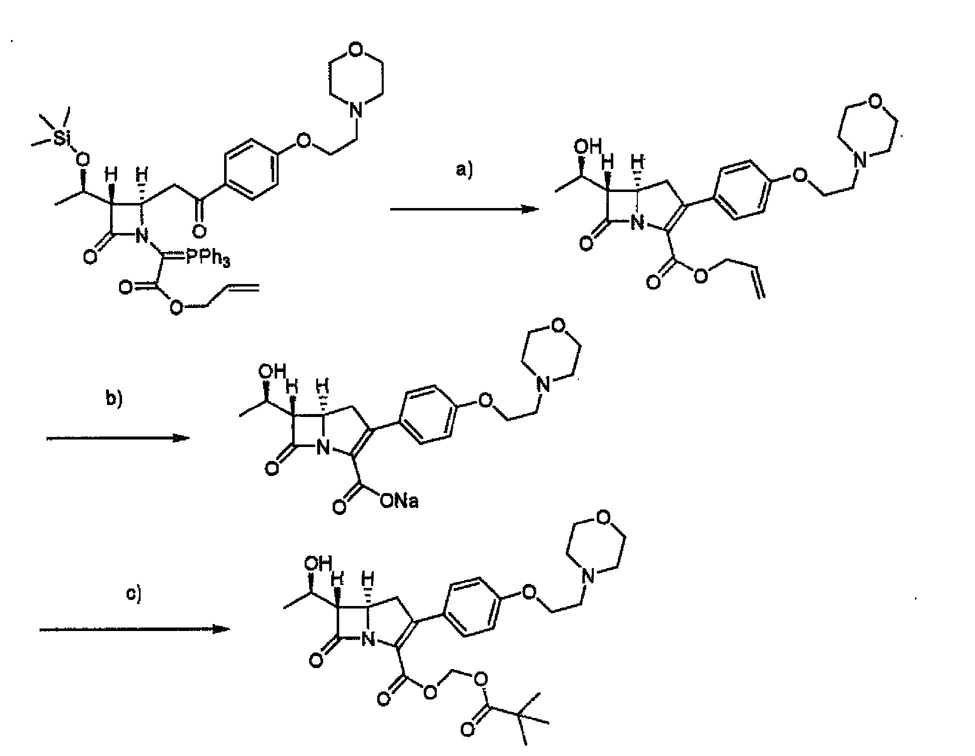

### Step a)

Allyl ((2R,3S)-2-{2-[4-(2-morpholin-4-ylethoxy)phenyl]-2-oxoethyl}-4-oxo-3-{(1R)-1-[(trimethylsilyl)oxy]ethyl}azetidin-1-yl)(triphenylphosphoranilidene) acetate (1.36g, 1.71 mmol) obtained by reference example 18 was dissolved in m-xylene (27 ml), and thereto were added N,O-bistrimethylsily-lacetamide (0.84 ml, 3.4 mmol) and hydroquinone (20 mg). The mixture was heated at 100°C for 1 hour and then refluxed under heating at 125°C for 4 hours. After being cooled, the reaction mixture was concentrated, and the residue was purified by silica gel column chromatography (silica gel 120 mL, chloroform : methanol = 100:0 - 100:4) to give an orange oil (0.92 g). This product without further purification was dissolved in acetonitrile (28 ml) and water (14 ml), and thereto was gradually dropped 0.1 N hydrochloric acid under cooling in an ice bath with a pH meter to adjust pH to 3.0. After 6 minutes, thereto was gradually dropped a saturated aqueous sodium hydrogen carbonate solution to adjust pH to 6.8, and saturated brine (50 ml) was added thereto. After extracting with ethyl acetate, the combined organic layer was dried over anhydrous sodium sulfate, filtered, and concentrated to give allyl (5R,6S)-6-[(1R)-1-hydroxyethyl]-3-[4-(2-morpholin-4-ylethoxy)phenyl]-7-oxo-1-azabicyclo[3.2.0]hepto-2-ene-2-carboxylate (0.70 g, yield 71%) as a brown oil. The product was used for next reaction without further purification.
¹H NMR (400 MHz, CDCl₃) δ 1.38 (d, 3 H, J = 6.4 Hz), 1.78 (br. s, 1 H), 2.48-2.71 (m, 4H), 2.75-2.96 (m, 2H), 3.17-3.32 (m, 3 H), 3.67-3.87 (m, 4H), 4.22-4.31 (m, 2 H), 4.62-4.68 (m, 1 H), 4.72-4.78 (m, 1 H), 5.21 (dd, J = 10.8, 1.6 Hz, 1 H), 5.33 (dd, J = 17.2, 1.6Hz, 1 H), 5.83-5.97 (m, 1 H), 6.87 (d, J = 6.8 Hz, 2 H), 7.37 (d, J = 8.8 Hz, 2 H).

### Step b)

Allyl (5R,6S)-6-[(1R)-1-hydroxyethyl]-3-[4-(2-morpholin-4-ylethoxy)phenyl]-7-oxo-1-azabicyclo[3.2.0]hepto-2-ene-2-carboxylate (0.27 g, 0.656 mmol) obtained in above step was dissolved under ice+cooling in a mixture of methylene chloride (12.4 ml) and ion-exchange water (24.8 ml). Thereto were added dichloro bis(triphenylphosphine)palladium(II) (21.4 mg, 0.031 mmol) and tri-n-butyltin hydride (3.37 ml, 12.2 mmol), followed by vigorously stirring for 10 minutes. Thereto were added sodium carbonate (102 mg, 0.962 mmol) and cooled ion-exchange water (12.4 ml), and the mixture was vigorously stirred for additional 5 minutes. The aqueous layer was separated from the reaction mixture, and extracted twice with ion-exchange water (5 ml). The extract was allowed to stand in vacuo under ice cooling for 45 minutes to remove the organic solvent. The residue was purified by C18 reverse-phase column chromatography (Wakosil C18 reverse-phase column, mobile phase; ion-exchange water/THF = 100:0~100:3) and further lyophilized to give sodium (5R,6S)-6-[(1R)-1-hydroxyethyl]-3-[4-(2-morpholin-4-ylethoxy)phenyl]-7-oxo-1-azabicyclo[3.2.0]hepto-2-ene-2-carboxylate (210 mg, yield 40%).
¹H NMR (400 MHz, D₂O) δ 1.16 (d, 3H, J=6.4Hz), 2.50-2.67 (m, 4H), 2.77 (t, 2H, J=5.2Hz), 2.96 (dd, 1H, J=17.2Hz, 1.0.4Hz), 3.31 (dd, 1H, J= 17.2Hz, 8.4Hz), 3.36-3.38 (m, 1H), 3.67 (t, 2H, J=8.8Hz), 4.10-4.23 (m, 4H), 6.84 (d, 2H, J=8.0Hz), 7.21 (d, 2H, J=8.0Hz).

### Step c)

Sodium (5R,6S)-6-[(1R)-1-hydroxyethyl]-3-[4-(2-morpholiil-4-ylethoxy)phenyl]-7-oxo-1-azabicyclo[3.2.0]hepto-2-ene-2-carboxylate (70 mg, 0.165 mmol) obtained by above step b) was dissolved in dried DMF (2.0 ml). Thereto were added benzyltriethylammonium chloride (131.5mg, 0.58 mmol) and then pivaloyloxymethyl chloride (87 mg, 0.58mmol), followed by stirring for 5 hours. To the reaction mixture were added ethyl acetate and cooled phosphate buffer (pH 7.46), and the mixture was extracted and separated by a separating funnel. The organic layer was washed three times with cooled brine and dried over anhydrous sodium sulfate. After removal of the solvent in vacuo, the residue was purified by silica gel column chromatography (neutral silica gel 15 ml, chloroform : methanol = 100:0 ~ 100:5) to give [(2,2-dimethylpropanoyl)oxy]methyl (5R,6S)-6-[(1R)-1-hydroxyethyl]-3-[4-(2-morpholin-4-ylethoxy)phenyl]-7-oxo-1-azabicyclo[3.2.0]hepto-2-ene-2-carboxylate (84 mg, yield 99%) as a yellow amorphous.
¹H NMR (400 MHz, CDCl₃) δ 1.19 (s, 9H), 1.36 (d, 3H, J=6.0Hz), 2.50-2.65 (m, 4H), 2.81 (t, 2H, J=5.6Hz), 3.15-3.33 (m, 3H), 3.73 (t, 4H, J=4.8Hz), 4.12 (t, 2H, J=6.4Hz), 4.23-4.32 (m, 2H), ,5.79 (d, 1H, J=6.4Hz), 5.88 (d, 1H, J=6.4Hz), 6.84 (d, 2H, J=8.4Hz), 7.35 (d, 2H, J=11.2Hz).

### Reference example 1

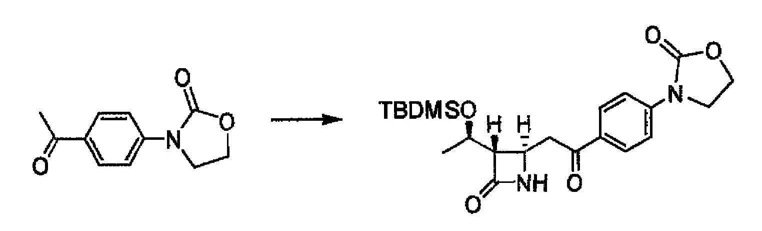

To a solution of 4-acetylphenyl-1-(1,3-oxazolidin-2-one) (1.71 g, 8.3 mmol) and triethylamine (0.927g, 9.2 mmol) in methylene chloride (35 ml) was dropped at 0°C trimethylsilyl trifluoromethanesulfonate (1.85 g, 8.3 mmol), followed by stirring for 20 minutes. Further triethylamine (0.337g, 3.7 mmol) and trimethylsilyl trifluoromethanesulfonate (0.556 g, 3.0 mmol) were dropped thereto, and the mixture was stirred for 20 minutes. To the reaction mixture were added under ice cooling (2R,3R)-3-((1R)-1-{[tert-butyl(dimethyl)silyl]oxy}ethyl)-4-oxo-2-azetidinyl acetate (2.4 g, 8.3 mmol), zinc iodide(1.6 g, 5.0 mmol), and the mixture was stirred at room temperature for 1 hour and 20 minutes. To the reaction mixture were added ethyl acetate and saturated brine, and the mixture was extracted, separated by a separating funnel, dried over anhydrous sodium sulfate, filtered and concentrated. The residue was purified by silica gel column chromatography (silica gel 50 g, ethyl acetate: hexane = 1:3 ~ ethyl acetate only) to give 3-(4-{[(2R,3S)-3-((1R)-1-{[tert-butyl(dimethyl)silyl]oxy}ethyl)-4-oxoazetidin-2-yl]acetyl}phenyl)-1,3-oxazolidin-2-one (2.6 g, yield 71%).
¹H NMR (400 MHz, CDCl₃) δ 0.02-0.13 (m, 6 H), 0.87 (s, 9 H), 1.25 (d, 3 H, J = 6.1 Hz), 2.88 (br-s, 1 H), 2.92 (s, 3 H), 3.07-3.17 (m, 1 H), 3.36-3.44 (m, 1 H), 3.50-3.58 (m, 2 H), 3.82-3.89 (m, 2 H), 4.04-4.13 (m, 1 H), 4.14-4.25 (m, 1 H), 6.13 (s, 1 H), 7.67 (d, 2 H, J = 8.9 Hz), 7.94 (d, 2 H, J = 8.9 Hz). Reference example 2

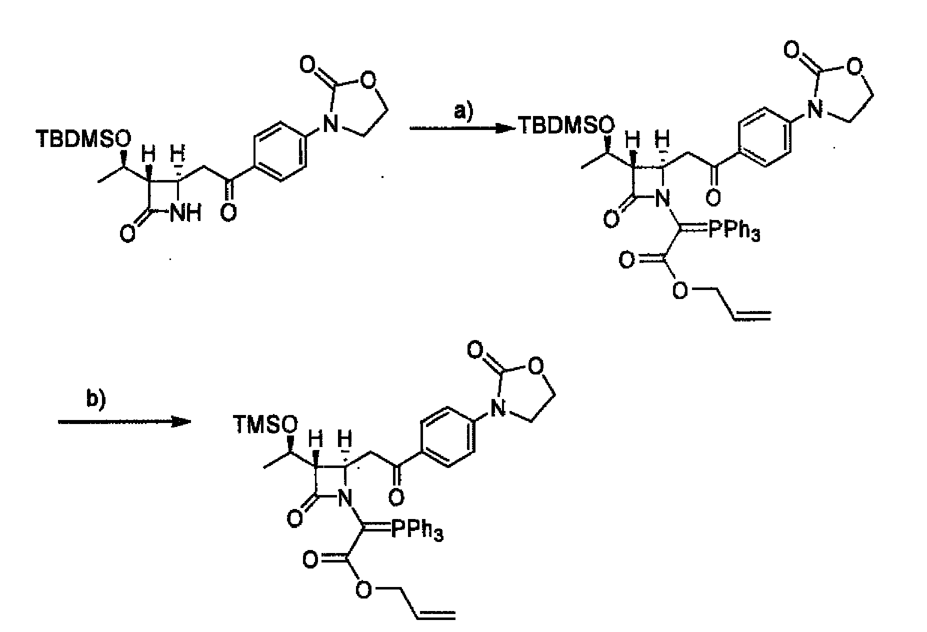

### Step a)

To a solution of 3-(4-{[(2R,3S)-3-((1R)-1-{[tert-butyl(dimethyl)silyl]oxy}ethyl)-4-oxoazetidin-2-yl]acetyl}phenyl)-1,3-oxazolidin-2-one (2.6 g, 5.9 mmol) obtained in reference example 1 in toluene (60 ml) was dropped at room temperature allyl dihydroxyacetate (1.17 g, 8.9 mmol), and then the mixture was stirred at 120°C for 4 hours during azeotropic distillation with Dean-Stark evaporator. The reaction mixture was concentrated and the residue was dissolved in THF (50 ml). Thereto were added at -20°C 2,6-lutidine (1.02 g, 9.5 mmol) and thionyl chloride (0.984 g, 8.3 mmol), and then added at room temperature THF (20 ml), followed by stirring for 1 hour. The reaction mixture was filtered and concentrated. The residue was dissolved in 1,4-dioxane (50 ml) and thereto were added at room temperature 2,6-lutidine (1.40 g, 13 mmol) and triphenylphosphine (2.79 g, 11 mmol), followed by stirring at 50°C for 6 hours. To the reaction mixture was added a saturated aqueous sodium hydrogen carbonate solution, and the mixture was extracted with ethyl acetate and separated by a separating funnel. The organic layer was washed with saturated brine, dried over anhydrous sodium sulfate, filtered and concentrated. The residue was purified by silica gel column chromatography (silica gel 70g, ethyl acetate: hexane = 1:2 ~ ethyl acetate) to give allyl ((3S,4R)-3-((1R)-1-{[tert-butyl(dimethyl)silyl]oxy}ethyl)-2-oxo-4-{2-oxo-2-[4-(2-oxo-1,3-oxazolidin-3-yl)phenyl]ethyl}azetidin-1-yl)(triphenylphosphoranilidene) acetate (3.51 g, yield 75%).
LC/MS (EI) 791.32 (M+1)

### Step b)

Allyl ((3S,4R)-3-((1R)-1-{[tert-butyl(dimethyl)silyl]oxy}ethyl)-2-oxo-4-{2-oxo-2-[4-(2-oxo-1,3-oxazolidin-3-yl)phenyl]ethyl}azetidin-1-yl)(triphenylphosphoraliilidene) acetate (2.03 g) obtained in step a) was dissolved at room temperature in an aqueous 50% trifluoroacetic acid solution (50 ml). To the reaction mixture was added ethyl acetate (50 ml) and the mixture was washed with saturated brine (100 ml), a saturated aqueous sodium hydrogen carbonate solution (100 ml), dried over sodium sulfate, filtered and concentrated. The residue and triethyamine (1.3 g, 13 mmol) were dissolved in THF (50 ml), and thereto was added at 0°C chlorotrimethylsilane (0.98 g, 9 mmol), followed by stirring for 30 minutes. To the reaction mixture was added ethyl acetate and the mixture was washed with a saturated aqueous sodium hydrogen carbonate solution (50 ml) and saturated brine (100 ml), dried over anhydrous sodium sulfate, filtered and concentrated. The residue was purified by silica gel column chromatography (silica gel 30 g, acetone : chloroform = 1:1) to give allyl ((3S,4R)-2-oxo-4-{2-oxo-2-[4-(2-oxo-1,3-oxazolidin-3-yl)phenyl]ethyl}-3-{(1R)-1-[{trimethylsilyl)oxy]ethyl}azetidin-1-yl)(triphenylphosphoranilidene) acetate (1.67 g, yield 87%).
LC/MS (El) 749.27 (M+1)

### Reference example 3

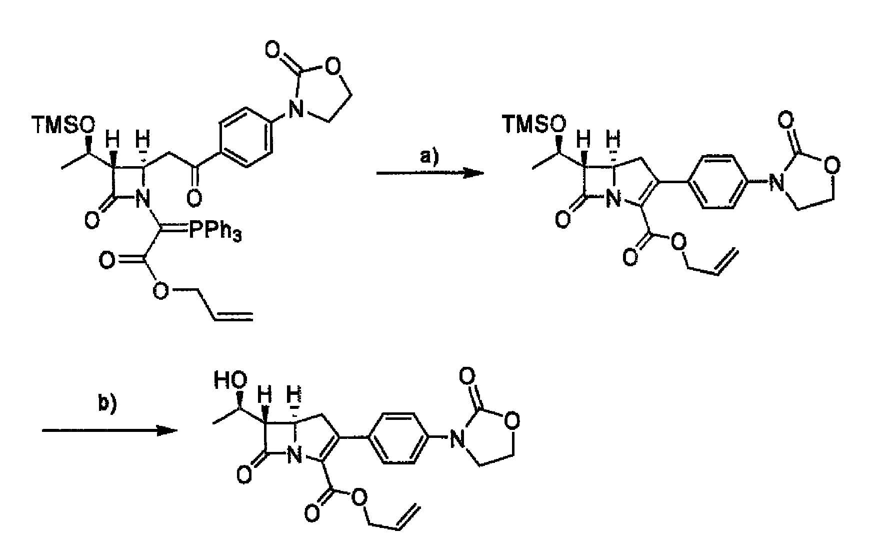

### Step a)

Allyl ((3S,4R)-2-oxo-4-{2-oxo-2-[4-(2-oxo-1,3-oxazolidin-3-yl)phenyl]ethyl}-3-{(1R)-1-[(trimethylsilyl)oxy]ethyl}azetidin-1-yl)(triphenylphosphoranilidene) acetate (1.67 g) obtained by reference example 2 was dissolved in toluene (100 ml), and thereto were added N,O-bistrimethylsilylacetamide (0.91 g) and 2,6-di-tert-butyl-4-methylphenol (20 mg), followed by refluxing under heating for 8 hours. After being cooled, the reaction mixture was concentrated and the residue was purified by silica gel column chromatography (silica gel 50 g, ethyl acetate: hexane = 1:2 ~ 2:1) to give allyl (5R,6S)-7-oxo-3-[4-(2-oxo-1,3-oxazolidin-3-yl)phenyl]-6-{(1R)-1-[(trimethylsilyl)oxy]ethyl}-1-azabicyclo[3.2.0]hept-2-ene-2-carboxylate (0.55 g, yield 53%).
¹H NMR (400 MHz, CDCl₃) δ 0.14 (s, 9 H), 1.29 (d, 3 H, J = 6.2 Hz), 3.12-3.33 (m, 3 H), 4.02-4.09 (m, 2 H), 4.13-4.23 (m, 2 H), 4.45-4.55 (m, 2 H), 4.61-4.80 (m, 2 H), 5.14-5.22 (m, 1 H), 5.29-5.38 (m, 1 H), 5.83-5.99 (m, 1 H), 7.41 (d, 2 H, J = 8.4 Hz), 7.56 (d, 2 H, J = 8.4 Hz).

### Step b)

Allyl (5R,6S)-7-oxo-3-[4-(2-oxo-1,3-oxazolidin-3-yl)phenyl]-6-{(1R)-1-[(trimethylsilyl)oxy]ethyl}-1-azabicyclo[3.2.0]hept-2-ene-2-carboxylate (0.55 g, 1.2 mmol) obtained by step a) was dissolved in THF (55 ml) and water (5 ml), and to the solution was gradually dropped 1 N hydrochloric acid under cooling in an ice bath with a pH meter to adjust pH to 2.5. After 10 minutes, thereto were gradually dropped a saturated aqueous sodium hydrogen carbonate solution (40 ml) and saturated brine (40 ml). After extracting with ethyl acetate (50ml), the organic layer was dried over anhydrous sodium sulfate, filtered, and concentrated to give allyl (5R,6S)-6-[(1R)-1-hydroxyethyl]-7-oxo-3-[4-(2-oxo-1,3-oxazolidin-3-yl)phenyl]-1-azabicyclo[3.2.0]hept-2-ene-2-carboxylate (0.49 g, 1.2 mmol, yield 100%).
¹H NMR (400 MHz, CDCl₃) δ 1.37 (d, 3 H, J = 6.3 Hz), 1.78 (d, 1 H, J = 4.8 Hz), 3.14-3.31 (m, 3 H), 3.98-4.06 (m, 2 H), 4.19-4.35 (m, 2 H), 4.43-4.56 (m, 2 H), 4.59-4.78 (m, 2 H), 5.21 (d, 1 H, J = 9.2 Hz), 5.33 (d, 1 H, J = 17.1 Hz), 5.82-5.93 (m, 1 H), 7.41 (d, 2 H, J = 8.4 Hz), 7.55 (d, 2 H, J = 8.4 Hz).

### Reference example 4

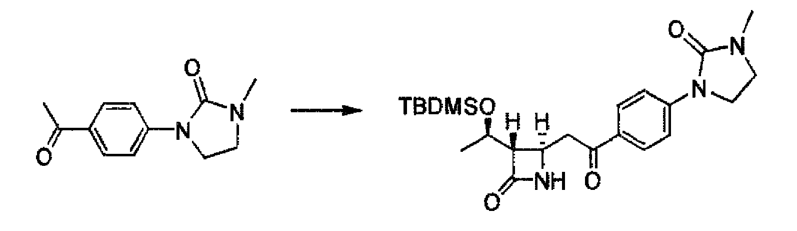

To a solution of 4-acetylphenyl-1-(3-methyl-1,3-imidazolidin-2-one) (2.42 g, 11 mmol) and triethylamine (1.6 g, 16 mmol) in methylene chloride (60 ml) was dropped at 0°C trimethylsilyl trifluoromethanesulfonate (3.2 g, 14 mmol), and the mixture was stirred for 20 minutes. To the reaction mixture were added under ice cooling (2R,3R)-3-((1R)-1-{[tert-butyl(dimethyl)silyl]oxy}ethyl)-4-oxo-2-azetidinyl acetate(3.2 g, 11 mmol) and zinc iodide (2.1 g, 6.7 mmol). After the mixture was stirred at room temperature for 1 hour, thereto was added zinc iodide (1.0 g, 3.3 mmol) and the mixture was stirred at room temperature for 30 minutes. To the reaction mixture were added a 5% potassium hydrogen sulfate solution and ethyl acetate, and the mixture was extracted and separated by a separating funnel. The extract was dried over anhydrous sodium sulfate, filtered and concentrated. The residue was purified by silica gel column chromatography (silica gel 120 g, ethyl acetate: hexane = 1:1 - ethyl acetate only) to give 1-(4-{[(2R,3S)-3-((1R)-1-{[tert-butyl(dimethyl)silyl]oxy}ethyl)-4-oxoazetidin-2-yl]acetyl}phenyl)-3-methylimidazolidin-2-one (4.2 g, yield 76%).
¹H NMR (400 MHz, CDCl₃) δ 0.07 (s, 6 H), 0.86 (s, 9 H), 1.24 (d, 3 H, J = 6.1 Hz), 2.88 (br. s, 1 H), 2.92 (s, 3 H), 3.02-3.13 (m, 1 H), 3.38-3.48 (m, 1 H), 3.51-3.58 (m, 2 H), 3.81-3.89 (m, 2 H), 4.11 (d, 1 H, J = 9.9 Hz), 4.12-4.23 (m, 1 H), 6.13 (s, 1 H), 7.67 (d, 2 H, J = 8.9 Hz), 7.92 (d, 2 H, J = 8.9 Hz).

### Reference example 5

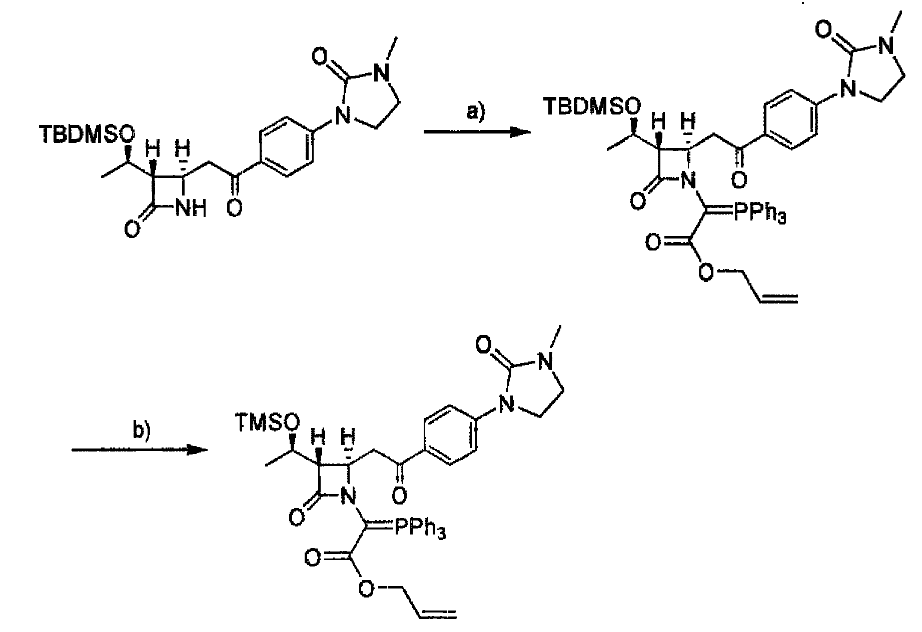

### Step a)

To a solution of 1-(4-{[(2R,3S)-3-((1R)-1-{[tert-butyl(dimethyl)silyl]oxy}ethyl)-4-oxoazetidin-2-yl)acetyl}phenyl)-3-methylimidazolidin-2-one (2.2 g, 4.9. mmol) obtained by reference example 4 in toluene (60 ml) was dropped at room temperature allyl dihydroxyacetate (0.98 g, 7.4 mmol), an.d then the mixture was stirred 120°C for 4 hours during azeotropic distillation with Dean Stark evaporator. The reaction mixture was concentrated and the residue was dissolved in THF (60 ml). Thereto were added at -20°C 2,6-lutidine (0.85 g, 7.9 mmol) and thionyl chloride (0.82 g, 6.9 mmol), followed by adding at room temperature THF (20 ml). After stirring for 1 hour, the reaction mixture was filtered and concentrated. The residue was dissolved in 1,4-dioxane (60 ml) and thereto were added at room temperature 2,6-lutidine (1.16 g, 11 mmol) and triphenylphosphine (2.33 g, 8.9 mmol), followed by stirring at 50°C for 6 hours. To the reaction mixture was added a saturated aqueous sodium hydrogen carbonate solution, and the mixture was extracted with ethyl acetate and separated by a separating funnel. The organic layer was washed with saturated brine (twice), dried over anhydrous sodium sulfate, filtered and concentrated. The residue was purified by silica gel column chromatography (silica gel 120 g, ethyl acetate: hexane = 1:1 ~ ethyl acetate only) to give allyl ((2R,3S)-3-((1R)-1-{[tert-butyl(dimethyl)silyl]oxy}ethyl)-2-{2-[4-(3-methyl-2-oxoimidazolidin-1-yl)phenyl]-2-oxoethyl}-4-oxoazetidin-1-yl)(triphenylphosphoranilidene) acetate (3.10 g, yield 78%).
LC/MS (EI) 804.35 (M+1)

### Step b)

Allyl ((2R,3S)-3-((1R)-1-{[tert-butyl(dimethyl)silyl]oxy}ethyl)-2-{2-[4-(3-methyl-2-oxoimidazolidin-1-yl)phenyl]-2-oxoethyl}-4-oxoazetidin-1-yl)(triphenylphosphoranilidene) acetate (3.1 g) obtained by step a) was dissolved at room temperature in a 50% aqueous trifluoroacetic acid solution (30 ml). To the reaction mixture was added ethyl acetate (30 ml) and the mixture was washed with saturated brine (70 ml) and a saturated aqueous sodium hydrogen carbonate solution. (70 ml), successively, dried over anhydrous sodium sulfate, filtered and concentrated. The residue and triethylamine (2.0 g, 19 mmol) were dissolved in THF (50 ml) and thereto was added at 0°C chlorotrimethylsilane (1.5 g, 14 mmol), followed by stirring for 1 hour. The reaction mixture was diluted with ethyl acetate, washed with a saturated aqueous sodium hydrogen carbonate solution (50 ml) and saturated brine (50 ml), successively, dried over anhydrous sodium sulfate, filtered and concentrated. The residue was purified by silica gel column chromatography (silica gel 120 g, ethyl acetate only - acetone only) to give allyl ((2R,3S)-2-{2-[4-(3-methyl-2-oxoimidazolidin-1-yl)phenyl]-2-oxoethyl}-4-oxo-3-{(1R)-1-[(trimethylsilyl)oxy]ethyl}azetidin-1-yl)(triphenylphosphoranilidene)acetate (2.11 g, yield 72%).
LC/MS (EI) 762.31 (M+1)

### Reference example 6

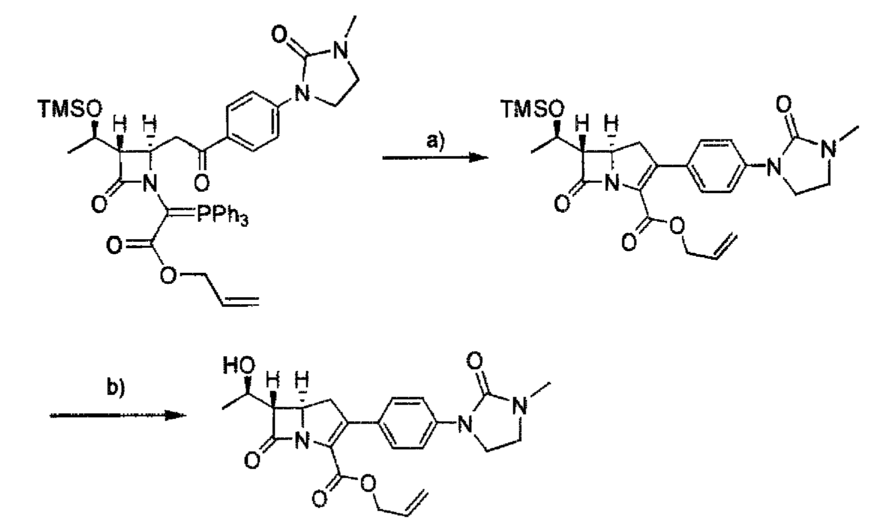

### Step a)

Allyl ((2R,3S)-2-{2-[4-(3-methyl-2-oxoimidazolidin-1-yl}phenyl]-2-oxoethyl}-4-oxo-3-{(1R)-1-[(trimethylsilyl)oxy]ethyl}azetidin-1-yl)(triphenylphosphoranilidene)acetate (2.11 g) obtained by reference example 5 was dissolved in toluene (150 ml), and thereto were added N,O-bistrimethylsilylacetamide (1.37 ml) and 2,6-di-tert-butyl-4-methylphenol (20 mg). After refluxing under heating for 12 hours, thereto was added m-xylene (15 ml), and the mixture was refluxed under heating for 5 hours. After being cooled, the reaction mixture was concentrated and the residue was purified by silica gel column chromatography (silica gel 70 g, ethyl acetate: hexane = 1:1 - 2:1) to give allyl (5R,6S)-3-[4-(3-methyl-2-oxoimidazolidin-1-yl)phenyl]-7-oxo-6-{(1R)-1-[(trimethylsilyl)oxy]ethyl}-1-azabicyclo[3.2.0]hept-2-ene-2-carboxylate (1.00 g, yield 75%).
¹H NMR (400 MHz, CDCl₃) δ 0.14 (s, 9 H), 1.34 (d, 3 H, J = 6.2 Hz), 2.95 (s, 3 H), 3.18-3.35 (m, 3 H), 3.50-3.58 (m, 2 H), 3.82-3.90 (m, 2 H), 4.17-4.32 (m, 2 H), 4.65-4.85 (m, 2 H), 5.25 (d, 1 H, J = 9.2 Hz), 5.38 (d, 1 H, J = 15.7 Hz), 5.86-6.00 (m, 1 H), 7.44 (d, 2 H, J = 8.9 Hz), 7.59 (d, 2 H, J = 8.9 Hz).

### Step b)

Allyl (5R,6S)-3-[4-(3-methyl-2-oxoimidazolidin-1-yl)phenyl]-7-oxo-6-{(1R)-1-[(trimethylsilyl)oxy]ethyl}-1-azabicyclo[3.2.0]hept-2-ene-2-carboxylate (0.66 g, 0.80 mmol) obtained step a) was dissolved in THF(45 ml) and water (5 ml), and to the solution was gradually dropped 0.1 N hydrochloric acid under cooling in an ice bath with a pH meter to adjust pH to 3.0. After 30 minutes, thereto were gradually dropped a saturated aqueous sodium hydrogen carbonate solution (40 ml) and saturated brine (40 ml). The mixture was extracted with ethyl acetate (50 ml). The organic layer was dried over anhydrous sodium sulfate, filtered and concentrated to give allyl (5R,6S)-6-[(1R)-1-hydroxyethyl]-3-[4-(3-methyl-2-oxoimidazolidin-1-yl)phenyl]-7-oxo-1-azabicyclo[3.2.0]hept-2-ene-2-carboxylate (0.46 g, 1.0 mmol, yield 100%).
¹H NMR (400 MHz, CDCl₃) δ 1.37 (d, 3 H, J = 6.3 Hz), 1.85 (br-s, 1 H), 2.90 (s, 3 H), 3.17-3.35 (m, 3 H), 3.43-3.57 (m, 2 H), 3.78-.3.84 (m, 2 H), 4.21-4.30 (m. 2 H), 4.62-4.78 (m, 2 H), 5.20 (d, 1 H, J = 9.2 Hz), 5.32 (d, 1 H, J = 15.7 Hz), 5.80-5.95 (m. 1 H), 7.32-7.41 (m, 2 H), 7.53-7.59 (m, 2 H).

### Reference example 7

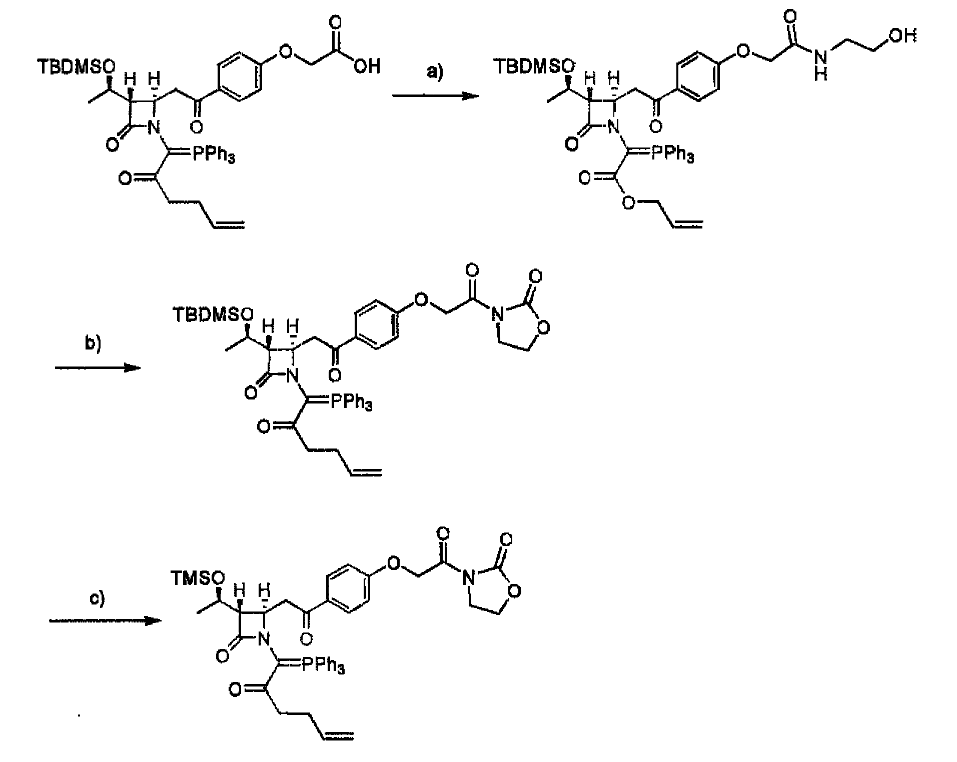

### Step a}

To a solution of 2-tert-butyldimethylsilyloxyethylamine (0.63 g, 3.6 mmol) in methylene chloride (30 ml) were dropped at 0°C 4-dimethylaminopyridine (26 mg, 0.21 mmol), 1-ethyl-3-(3-dimethylaminopropyl)carbodiimide (0.90 g, 4.7 mmol), and 4-{[(2R,3S)-1-[2-(allyloxy)-2-oxo-1-(triphenylphosphoranilidene)ethyl]-3-((1R)-1-{[tert-butyl(dimethyl)silyl]oxy}ethyl)-4-oxoazetidin-2-yl]acetyl}phenoxy)acetic acid (1.7 g, 2.1 mmol) in methylene chloride (30 ml). The mixture was stirred at room temperature for 4 hours. To the reaction mixture was added a saturated aqueous sodium hydrogen carbonate solution, and the mixture was extracted with ethyl acetate and separated by a separating funnel. The organic layer was washed with saturated brine, dried over anhydrous sodium sulfate, filtered and concentrated. The residue was purified by silica gel column chromatography (silica gel 110 g, ethyl acetate: hexane = 2:1 ~ 5:1) to give allyl {(2R,3S)-3-((1R)-1-{[tert-butyl(dimethyl)silyl]oxy}ethyl)-2-[2-(4-{2-[(2-{[tert-butyl(dimethyl)silyl]oxy}ethyl)amino]-2-oxoethoxy}phenyl)-2-oxoethyl]-4-oxoazetidin-1-yl}(triphenylphosphoranilidene)acetate (1.1 g, yield 56%). This product was dissolved in THF (30 ml) and thereto were dropped at 0°C acetic acid (71 mg, 1.2 mmol) and 1.0 M tetra-n-butylammonium fluoride/THF (1.18 ml, 1.2 mmol), followed by stirring at the same temperature for 24 hours. Further thereto were dropped at 0°C acetic acid (71 mg, 1.2 mmol) and 1.0 M tetra-n-butylammoxrium fluaride/THF (1.1.8 ml, 1.2 mmol) and the mixture was stirred for 8 hours. To the reaction mixture was added a saturated aqueous sodium hydrogen carbonate solution and the mixture was extracted with ethyl acetate and separated by a separating funnel. The organic layer was washed with saturated brine, dried over anhydrous sodium sulfate, filtered and concentrated to give allyl {(2R,3S)-3-((1R)-1-{[tert-butyl(dimethyl)silyl]oxy}ethyl)-2-[2-(4-{2-[(2-hydroxyethyl)amino]-2-oxoethoxy}phenyl)-2-oxoethyl]-4-oxoazetidin-1-yl}(triphenylphasphoranilidene) acetate (1.0 g, yield 100%).
LC/MS (EI) 823.35 (M+1)

### Step b)

To a solution of allyl {(2R,3S)-3-((1R)-1-{[tert-butyl(dimethyl)silyl]oxy}ethyl)-2-[2-(4-{2-[(2-hydroxyethyl)amino]-2-oxoethoxy}phenyl)-2-oxoethyl]-4-oxoazetidin-1-yl}(triphenylphosphoranilidene) acetate (1.0 g) obtained by step a) in THF (30 ml) were added 1, 1'-carbonyldiimidazole (0.19 g, 1.2 mmol) and triethylamine (0.24 g, 2.4 mmol), and the mixture was stirred at room temperature for 3 days. Further thereto were added 1,1'-carbonyldiimidazole (0.19 g, 1.2 mmol) and triethylamine (0.24 g, 2.4 mmol) and the mixture was stirred at room temperature for 5 days. To the reaction mixture was added ethyl acetate (30 ml) and the mixture was washed with saturated brine (40 ml) and a saturated aqueous sodium hydrogen carbonate solution (40 ml), dried over sodium sulfate, filtered and concentrated to give allyl [(3S,4R)-3-((1R)-1-{[tert-butyl(dimethyl)silyl]oxy}ethyl)-2-oxo-4-(2-oxo-2-{4-[2-oxo-2-(2-oxo-1,3-oxazolidin-3-yl)ethoxy]phenyl}ethyl) azetidin-1-yl](triphenylphosphoranilidene)acetate (1.0 g, yield 100%).
LC/MS (EI) 849.33 (M+1)

### Step c)

Allyl [(3S,4R)-3-((1R)-1-{[tert-butyl(dimethyl)silyl]oxy}ethyl)-2-oxo-4-(2-oxo-2-{4-[2-oxo-2-(2-oxo-1,3-oxazolidin-3-yl)ethoxy]phenyl}ethyl)azetidin-1-yl](triphenylphosphoranilidene)acetate (1.28 g) obtained by step b) was dissolved at room temperature in an aqueous 50% trifluoroacetic acid solution (35 ml). To the reaction mixture was added ethyl acetate (30 ml), and the mixture was washed with saturated brine (70 ml) and a saturated aqueous sodium hydrogen carbonate solution (70 ml), dried over anhydrous sodium sulfate, filtered and concentrated. The residue and triethylamine (0.76 g, 7.6 mmol) were dissolved in THF (60 ml) and thereto was added at 0°C chlorotrimethylsilane (0.57 g, 5.3 mmol), followed by stirring 1 hour. To the reaction mixture was added ethyl acetate and the mixture was washed with a saturated aqueous sodium hydrogen carbonate solution (50 ml) and saturated brine (50 ml), successively, dried over anhydrous sodium sulfate, filtered and concentrated. The residue was purified by silica gel column chromatography (silica gel 70 g, ethyl acetate: hexane = 1:1 ~ ethyl acetate only) to give allyl (3S,4R)-2-oxo-4-(2-oxo-2-{4-[2-oxo-2-(2-oxo-1,3-oxazolidin-3-yl)ethoxyl]phenyl}ethyl)-3-{(1R)-1-[(trimethylsilyl)oxy]ethyl}azetidin-1-yl)(triphenylphosphoranilidene)acetate (0.73 g, yield 60%).
LC/MS (EI) 807.28 (M+1)

### Reference example 8

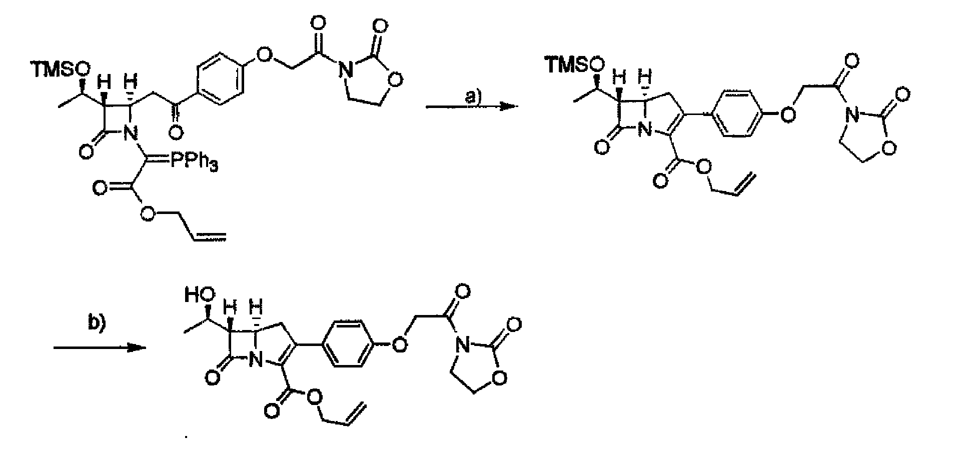

### Step a)

Allyl ((3S,4R)-2-oxo-4-(2-oxo-2-{4-[2-oxo-2-(2-oxo-1,3-oxazolidin-3-yl)ethoxy]phenyl}ethyl)-3-{(1R)-1-[(trimethylsilyl)oxy]ethyl}azetidin-1-yl)(triphenylphosphoranilidene)acetate (0.73 g) obtained by reference example 7 was dissolved in toluene (50 ml), and thereto were added N,O-bistrimethylsilylacetamide (0.37 g) and 2,6-di-tert-butyl-4-methylphenol (10 mg), followed by refluxing under heating for 6 hours. After being cooled, the reaction mixture was concentrated and the residue was purified by silica gel column chromatography (silica gel 33 g, ethyl acetate: hexane = 1:1 - ethyl acetate only) to give allyl (5R,6S)-7-oxo-3-{4-[2-oxo-2-(2-oxo-1,3-oxazolidin-3-yl)ethoxy]phenyl}-6-{(1R)-1-[(trimethylsilyl)oxy]ethyl}-1-azabieyclo[3.2.0]hept-2-ene-2-carboxylate (0.41 g, yield 89%).
¹H NMR (400 MHz, CDCl₃) δ 0.14 (s, 9 H), 1.29 (d, 3 H, J = 6.2 Hz), 3.08-3.27 (m, 3 H), 4.02-4.10 (m, 2 H], 4.1 l.-4.23 (m, 2 H), 4.52-4.59 (m, 2 H), 4.60-4.78 (m, 2 H), 5.19 (d, 1 H,J = 9.2 Hz), 5.25 (s, 2 H), 5.31 (d, 1 H, J = 15.7 Hz), 5.82-5.94 (m, 1 H), 6.91 (d, 2 H, J = 6.8 Hz), 7.35 (d, 2 H, J = 6.8 Hz).

### Step b)

Allyl (5R,6S)-7-oxo-3-{4-[2-oxo-2-(2-oxo-1,3-oxazolidin-3-yl)ethozy]phenyl}-6-{(1R)-1-[(trimethylsilyl)oxy]ethyl}-1-azabicyclo[3.2.0]hept-2-ene-2-carboxylate (0.41 g, 0.80 mmol) obtained by step a) was dissolved in THE (45 ml) and water (5 ml), and to the solution was gradually dropped 0.1 N hydrochloric acid under cooling in an ice bath with a pH meter to adjust pH to 3.0. After 30 minutes, thereto were gradually dropped a saturated aqueous sodium hydrogen carbonate solution (40 ml) and saturated brine (40 ml). The mixture was extracted with ethyl acetate (50 ml). The organic layer was dried over anhydrous sodium sulfate, filtered and concentrated to give allyl (5R,6S)-6-[(1R)-1-hydroxyethyl]-7-oxo-3-{4-[2-oxo-2-(2-oxo-1,3-oxazolidin-3-yl)ethoxy]phenyl}-1-azabicyclo[3.2.0]hept-2-ene-2-carboxylate (0.36 g, 0.80 mmol, yield 100%).
¹H NMR (400 MHz, CDCl₃) δ 1.37 (d, 3 H, J = 6.3 Hz), 1.73 (d, 1 H, 4.9 Hz), 3.12 -3.29 (m, 3 H), 4.02-4.10 (m, 2 H), 4.20-4.28 (m, 2 H), 4.52-4.59 (m, 2 H), 4.61-4.76 (m, 2 H), 5.20 (d, 1 H, J = 10.4 Hz), 5.25 (s, 2 H), 5.30 (d, 1 H, J = 17.2 Hz), 5.82-5.94 (m, 1 H), 6.87-6.92 (m, 2 H), 7.32-7.38 (m, 2 H).

### Reference example 9

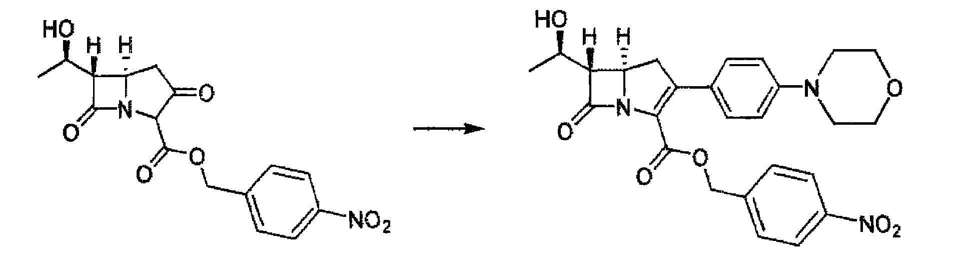

To a solution of 4-nitrobenzyl (5R,6S)-3,7-dioxo-6-{(1R)-1-hydroxyethyl}-1-azabicyclo[3.2.0]heptane-2-carboxylate (1.56 g) in methylene chloride (25 ml) were dropped at -78°C triethyamine (0.50 g, 4.9 mmol) and trifluoroacetic anhydride (1.33 g, 4.7 mmol). After stirring for 15 minutes, thereto were dropped at -78°C triethylamine (0.50 g, 4.9 mmol) and triethylsilyl trifluoromethanesulfonate (1.24 g, 4.7 mmol). Ten minutes later, thereto were added at -78°C 4-morpholin-4-ylphenyl-1-boric acid (0.93 g, 5.0 mmol) in THF (30 ml), potassium carbonate (1.86 g, 13 mmol) and tris(dibenzylidene acetone) dipalladium(0) (0.12 g, 0.13 mmol), followed by stirring at room temperature for 4 hours. The reaction mixture was filtered over Celite, and thereto was added water (30 ml). The mixture was extracted with ethyl acetate (30 ml), and the extract was washed with saturated brine (30 ml). The organic layer was dried over anhydrous sodium sulfate, filtered and concentrated. The residue was dissolved in THF (60 ml) and thereto were dropped at 0°C acetic acid (0.81 g, 13 mmol) and 1.0 M tetra-n-butylammonium fluoride/THF (1.18 ml, 13 mmol), followed by stirring at the same temperature for 1 hour. To the reaction mixture was added ethyl acetate (50 ml), and the mixture was washed with a saturated aqueous sodium hydrogen carbonate solution (50 ml) and saturated brine (50 ml), successively, dried over anhydrous sodium sulfate, filtered and concentrated. The residue was purified by silica gel column chromatography (silica gel 70 g, ethyl acetate: hexane = 1:2 ~ ethyl acetate only) to give 4-nitrobenzyl (5R,6S)-6-[(1R)-1-hydroxyethyl]-3-(4-morpholin-4-ylphenyl)-7-oxo-1-azabicyclo[3.2.0]hept-2-ene-2-carboxylate (1.3 g, yield 58%).
¹H NMR (400 MHz, CDCl₃) δ 1.39 (d, 3 H, J = 6.3 Hz), 1.74 (d, 1 H, 4.8 Hz), 3.12 -3.29 (m, 7 H), 3.80-3.87 (m, 4 H), 5.21 (d, 1 H, J = 13.8 Hz), 5.42 (d, 1 H, J = 13.8 Hz), 6.82 (d, 2 H, J = 8.9 Hz), 7.37 (d, 2 H, J = 6.9 Hz), 7.52 (d, 2 H, J = 8.8 Hz), 8.18 (d, 2 H, J = 8.8 Hz).

### Reference example 10

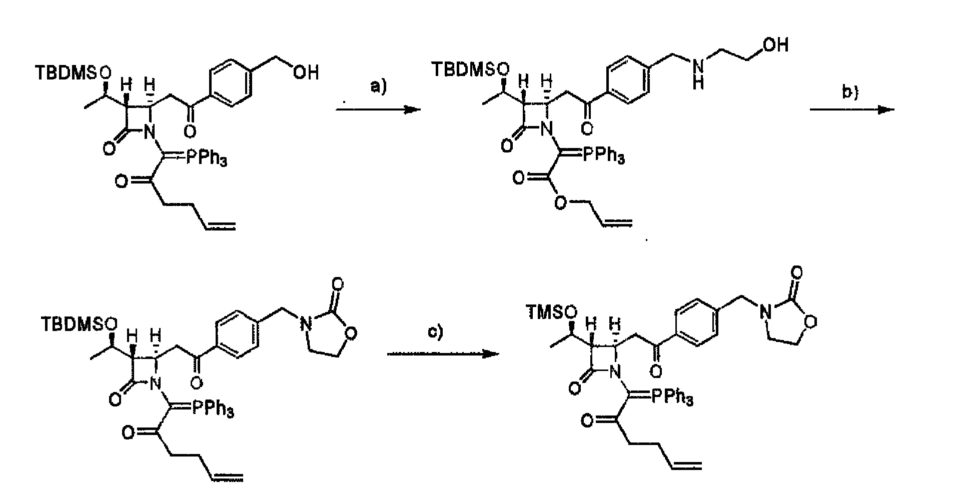

### Step a)

To a solution of allyl ((2R,3S)-3-((1R)-1-{[tert-butyl(dimethyl)silyl]oxy}ethyl)-2-{2-[4-(hydroxyethyl)phenyl]-2-oxoethyl}-4-oxoazetidin-1-yl)(triphenylphosphoranilidene)acetate (1.4 g,1.9 mmol) in methylene chloride (14 ml) was dropped at room temperature 1-chloro N,N,2-trimethyl-1-propenyl-amine (0.47 g, 1.9 mmol), followed by stirring for 1 hour. The reaction mixture was concentrated and the residue was dissolved in DMF (14 ml). Thereto were added at 0°C ethanolamine (0.58 g, 9.5 mmol), tetra-n-butylammonium bromide (1.2g, 3.8 mmol) and sodium hydrogen carbonate (0.40 g, 4.8 mmol), and the mixture was stirred at room temperature for 2 hours. To the reaction mixture was added a saturated aqueous ammonium chloride solution, and the mixture was extracted with ethyl acetate and separated by a separating funnel. The organic layer was washed with saturated brine, dried over anhydrous sodium sulfate, filtered and concentrated to give allyl {(2R,3S)-3-((1R)-1-{[tert-butyl(dimethyl)silyl]oxy}ethyl)-2-[2-(4-{[(2-hydroxyethyl)amino]methyl}phenyl)-2-oxoethyl]-4-oxoazetidin-1-yl}(triphenylphosphoranilidene)acetate (1.5 g). This product was used for next reaction without further purification.
LC/MS (EI) 779.36 (M+1)

### Step b)

To a solution of allyl {(2R,3S)-3-((1R)-1-{[tert-butyl(dimethyl)silyl]oxy}ethyl)-2-[2-(4-{[(2-hydroxyethyl)amino]methyl}phenyl)-2-oxoethyl]-4-oxoazetidin-1-yl}(triphenylphosphoranilidene)acetate (2.1 g) obtained by step a) in THF (35 ml) was added 1,1'-carbonyldiimidazole (0.70 g, 4.3 mmol). The mixture was stirred at room temperature for 2 hours. The reaction mixture was diluted with ethyl acetate (30 ml), washed with saturated brine (40 ml) and a saturated aqueous sodium hydrogen carbonate solution (40 ml), successively, dried over anhydrous sodium sulfate, filtered and concentrated. The residue was purified by silica gel column chromatography (silica gel 70 g, ethyl acetate: hexane = 1:1 ~ ethyl acetate only) to give allyl [(3S,4R)-3-((1R)-1-{[tert-butyl(dimethyl)silyl]oxy}ethyl)-2-oxo-4-(2-oxo-2-{4-[(2-oxo-1,3-oxazolidin-3-yl)methyl]phenyl}ethyl)azetidin-1-yl](triphenylphosphoranilidene)acetate (1.1 g, yield 52%).
LC/MS (El) 805.34 (M+1)

### Step c)

Allyl [(3S,4R)-3-((1R)-1-{[tert-butyl(dimethyl)silyl]oxy}ethyl)-2-oxo-4-(2-oxo-2-{4-[(2-oxo-1,3-oxazolidin-3-yl)methyl]phenyl}ethyl)azetidin-1-yl](triphenylphosphoranilidene)acetate (1.6 g) obtained by step b) was dissolved at room temperature in a 50% aqueous trifluoroacetic acid solution (36ml). The reaction mixture was diluted with ethyl acetate (30 ml). The mixture was washed with saturated brine (70 ml) and a saturated aqueous sodium hydrogen carbonate solution (70 ml), successively, dried over anhydrous sodium sulfate, filtered and concentrated. The residue and triethylamine (1.42 ml, 10 mmol) were dissolved in THF (60 ml) and thereto was added at 0°C chlorotrimethylsilane (0.91 ml, 7.1 mmol). After stirring for 40 minutes, thereto were further added triethylamine (0.71 ml, 5.0 mmol) and chlorotrimethylsilane (0.45 ml, 3.6 mmol), followed by stirring at 0°C for 30 minutes. The reaction mixture was diluted with ethyl acetate. The mixture was washed with a saturated aqueous sodium hydrogen carbonate solution (50 ml) and saturated brine (50 ml), successively, dried over anhydrous sodium sulfate, filtered and concentrated to give allyl ((3S,4R)-2-oxo-4-(2-oxo-2-{4-[(2-oxo-1,3-oxazolidin-3-yl)methyl]phenyl}ethyl)-3-{(1R)-1-[(trimethylsily)oxy]ethyl}azetidin-1-yl)(triphenylphosphoranilidene)acetate (1.55 g, yield 100%).
LC/MS (EI) 763.29 (M+1)

### Reference example 11

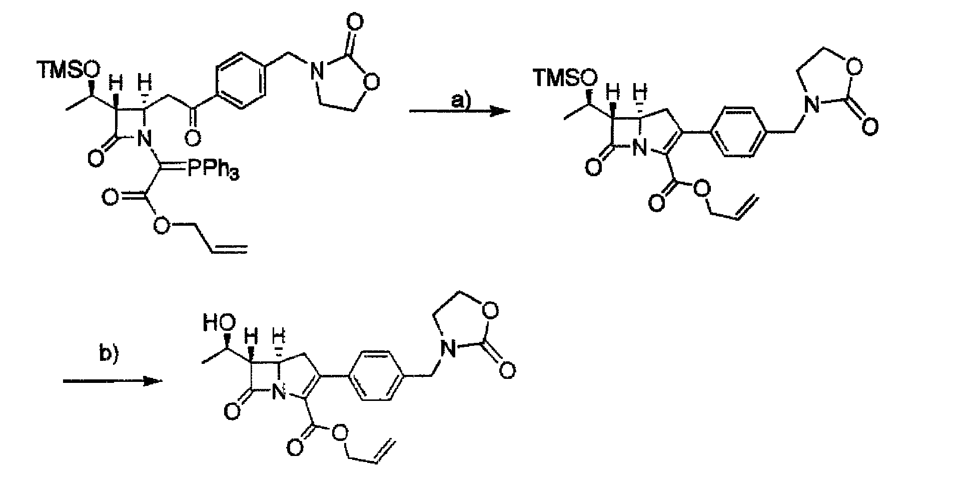

### Step a)

Allyl ((3S,4R)-2-oxo-4-(2-oxo-2-{4-[(2-oxo-1,3-oxazolidin-3-yl)methyl]phenyl}ethyl)-3-{(1R)-1-[(trimethylsilyl)oxy]ethyl}azetidin-1-yl)(triphenylphosphoranilidene)acetate (1.55 g) obtained by reference example 10 was dissolved in toluene (90 ml) and thereto were added N,O-bistrimethylsilylacetamide (1.01 ml) and hydroquinone (100 mg), followed by refluxing under heating for 6 hours. After being cooled, the reaction mixture was concentrated and the residue was purified by silica gel column chromatography (silica gel 70 g, ethyl acetate: hexane = 1:1 ~ 1:3) to give allyl (5R,6S)-7-oxo-3-{4-[(2-oxo-1,3-oxazolidin-3-yl)methyl]phenyl}-6-{(1R)-1-[(trimethylsilyl)oxy]ethyl}-1-azabicyclo[3.2.0]hept-2-ene-2-carboxylate (0.87 g, yield 88%).
¹H NMR (400 MHz, CDCl₃) δ 0.15 (s, 9 H), 1.29 (d, 3 H, J = 6.2 Hz), 3.11-3.32 (m, 3 H), 3.38-3.48 (m, 2 H), 4.16-4.28 (m, 2 H), 4.30-4.37 (m, 2 H), 4.42 (s, 2 H), 4.61-4.78 (m, 2 H), 5.18 (d, 1 H, J = 10,5 Hz), 5.30 (d, 1 H, J = 17.2 Hz), 5.81-5.96 (m, 1 H), 7.24-7,29 (m, 2 H), 7.33-7.39 (m, 2 H).

### Step b)

Allyl (5R,6S)-7-oxo-3-{4-[(2-oxo-1,3-oxazolidin-3-yl)methyl]phenyl}-6-{(1R)-1-[(trimethylsilyl)oxy]ethyl}-1-azabicyclo[3.2.0]hept-2-ene-2-carboxylate (0.87 g, 1.8 mmol) obtained by step a) was dissolved in THF (60 ml) and water (6 ml), and to the solution was gradually dropped 0.1 N hydrochloric acid under cooling in an ice bath with a pH meter to adjust pH to 3.0. After 30 minutes, thereto were gradually dropped a saturated aqueous sodium hydrogen carbonate solution (50 ml) and saturated brine (50 ml). The mixture was extracted with ethyl acetate (50 ml). The organic layer was dried over anhydrous sodium sulfate, filtered and concentrated to give allyl (5R,6S)-6-[(1R)-1-hydroxyethyl]-7-oxo-3-{4-[(2-oxo-1,3-oxazolidin-3-yl)methyl]phenyl}-1-azabicyclo[3.2.0]hept-2-ene-2-carboxylate (0.58 g, 1.4 mmol, yield 79%).
LC/MS (EI) 413.16 (M+1)

### Reference example 12

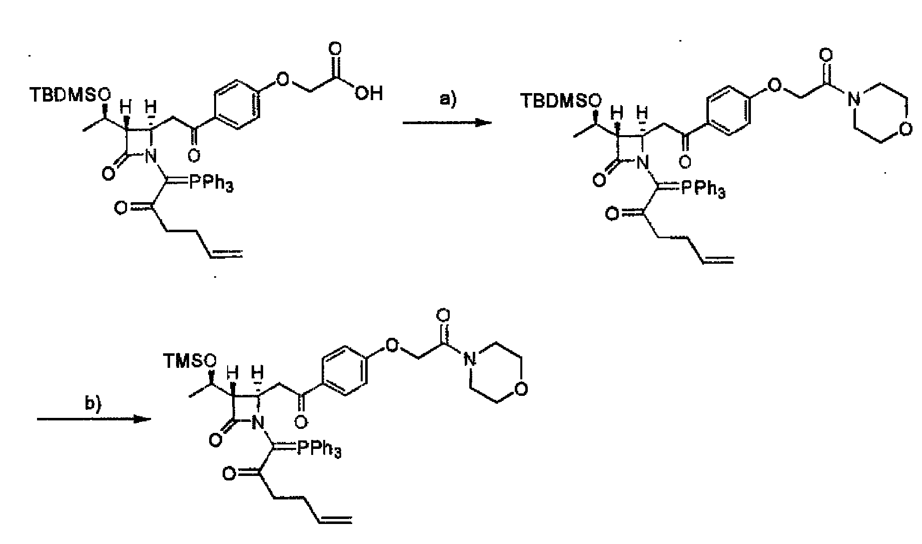

### Step a)

To a solution of morpholine (0.28 g, 3.2 mmol) in methylene chloride (30 ml) were dropped at 0°C 4-dimethylaminopyridine (23 mg, 0.19 mmol), 1-ethyl-3-(3-dimethylaminopropyl)carbodiimide (0.80 g, 4.2 mmol) and 4-{[(2R,3S)-1-[2-(allyloxy)-2-oxo-1-(triphenylphosphoranilidene)ethyl]-3-((1R)-1-{[tert-butyl(dimethyl)silyl]oxy}ethyl)-4-oxo-azetidin-2-yl]acetyl}phenoxy)acetic acid (1.5 g, 1.9 mmol) in methylene chloride (20 ml), followed by stirring at room temperature for 4 hours. To the reaction mixture was added a saturated aqueous sodium hydrogen carbonate solution and the mixture was extracted with ethyl acetate and separated by a separating funnel. The organic layer was washed with saturated brine, dried over anhydrous sodium sulfate, filtered and concentrated. The residue was purified by silica gel column chromatography (silica gel 70 g, chloroform : methanol = 1:0 - 20:1) to give allyl ((2R,3S)-3-((1R)-1-{[tert-butyl(dimethyl)silyl]oxy}ethyl)-2-{2-[4-(2-morpholin-4-yl-2-oxoethoxy)phenyl]-2-oxoethyl}-4-oxoazetidin-1-yl)(triphenylphosphoranilidene)acetate (1.3 g, yield 83%).
LC/MS (EI) 849.36 (M+1)

### Step b)

Allyl ((2R,3S)-3-((1R)-1-{[tert-butyl(dimethyl)silyl]oxy}ethyl)-2-{2-[4-(2-morpholin-4-yl-2-oxoethoxy)phenyl]-2-oxoethyl}-4-oxoazelidin-1-yl)(triphenylphosphoranilidene)acetate (1.3 g) obtained by Step a) was dissolved at room temperature in a 50% aqueous trifluoroacetic acid solution (15 ml). The reaction mixture was diluted with ethyl acetate (30 ml). The mixture was washed with saturated brine (50 ml) and a saturated aqueous sodium hydrogen carbonate solution (50 ml), successively, dried over anhydrous sodium sulfate, filtered and concentrated. The residue and triethylamine (0.80 g, 7.9 mmol) were dissolved in THE (30 ml) and thereto was added at 0°C chlorotrimethylsilane (0.60 g, 5.5 mmol). After stirring for 20 minutes, thereto were further added at 0°C triethylamine (0.40 g, 4.0 mmol) and chlorotrimethylsilane (0.30 g, 2.8 mmol), followed by stirring for 20 minutes. The reaction mixture was diluted with ethyl acetate. The mixture was washed with a saturated aqueous sodium hydrogen carbonate solution (50 ml) and saturated brine (50 ml), successively, dried over anhydrous sodium sulfate, filtered and concentrated to give allyl ((2R,3S)-2-[2-[4-(2-morpholin-4-yl-2-oxoethoxy)phenyl]-2-oxoethyl]-3-[(1R)-1-[(trimethylsilyl)oxy]ethyl]azetidin-1-yl)(triphenylphosphoranilidene)acetate (1.21 g, yield 95%).
LC/MS (EI) 807.32 (M+1)

### Reference example 13

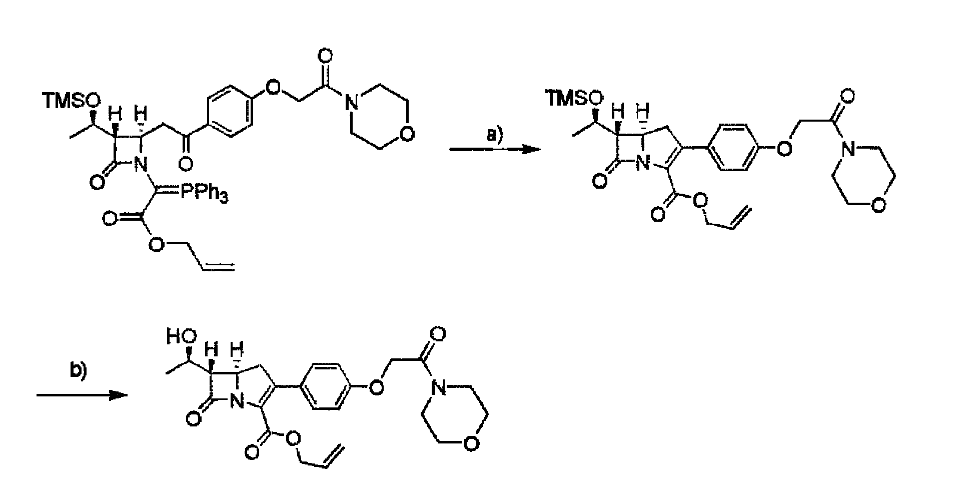

### Step a)

Allyl ((2R,3S)-2-[2-[4-(2-morpholin-4-yl-2-oxoethoxy)phenyl]-2-oxoethyl]-3-[(1R)-1-[(trimethylsilyl)oxy]ethyl]azetidin-1-yl)(triphenylphosphoranilidene)acetate (1.21 g) obtained by reference example 12 was dissolved in m-xylene (70 ml), and thereto were added N,O-bistrimethylsilylacetamide (0.74 ml) and 2,6-di-tert-butyl-4-methylphenol (10 mg), followed by refluxing under heating for 9 hours. After being cooled, the reaction mixture was concentrated and the residue was purified by silica gel column chromatography (silica gel 70 g, ethyl acetate: hexane = 1:1 ~ ethyl acetate only) to give allyl (5R,6S)-3-[4-(2-morpholin-4-yl-2-oxoethoxy)phenyl]-7-oxo-6-{(1R)-1-[(trimethylsilyl)oxy]ethyl}-1-azabicyclo[3.2.0]hept-2-ene-2-carboxylate (0.70 g, yield 88%).
¹H NMR (400 MHz, CDCl₃) δ 0.14 (s, 9 H), 1.29 (d, 3 H, J = 6.2 Hz), 3.12-3.28 (m, 3 H), 3.55-3.73 (m, 8 H), 4.17-4.26 (m, 2 H), 4.60-4.79 (m, 4 H), 5.20 (d, I H, J = 10.5 Hz), 5.33 (d, 1 H, J = 17.2 Hz), 5.82-5.96 (m, 1 H), 6.91 (d, 2 H, J = 8.9 Hzj, 7.37 (d, 2 H, J = 8.9 Hz).

### Step b)

Allyl (5R,6S)-3-[4-(2-morpholin-4-yl-2-oxoethoxy)phenyl]-7-oxo-6-{(1R)-1-[(trimethylsilyl)oxy]ethyl}-1-azabicyclo[3.2.0]hept-2-ene-2-carboxylate (0.70 g, 1.3 mmol) obtained by step a) was dissolved in THF (45 ml) and water (5 ml), and to the solution was gradually dropped 0.1 N hydrochloric acid under cooling in an ice bath with a pH meter to adjust pH to 3.0. After 30 minutes, thereto were gradually dropped a saturated aqueous sodium hydrogen carbonate solution (40 ml) and saturated brine (40 ml). The mixture was extracted with ethyl acetate (50 ml). The organic layer was dried over anhydrous sodium sulfate, filtered and concentrated to give allyl (5R,6S)-6-[(1R)-1-hydroxyethyl]-3-[4-(2-morpholin-4-yl-2-oxoethoxy)phenyl]-7-oxo-1-azabicyclo[3.2.0]hept-2-ene-2-carboxylate (0.59 g, 1.3 mmol, yield 97%).
¹H NMR (400 MHz, CDCl₃) δ 1.37 (d, 3 H, J = 6.3 Hz), 1.88 (br. s, 1 H), 3.13-3.32 (m, 3 H), 3.56-3.72 (m, 8 H), 4.20-4.31 (m, 2 H), 4.60-4.81 (m, 4 H), 5.20 (d, 1 H, J = 10.5 Hz), 5.32 (d, 1 H, J = 17.2 Hz), 5.82-5.96 (m, 1 H), 6.91 (d, 2 H, 8.9 Hz), 7.37 (d, 2 H, 8.9 Hz).

### Reference example 14

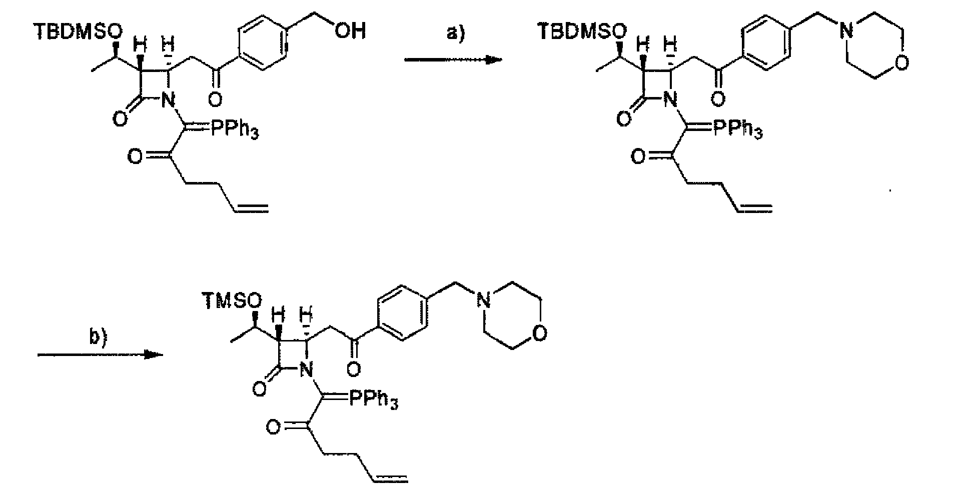

### Step a)

To a solution of allyl ((2R,3S)-3((1R)-1-{[tert-butyl(dimethyl)silyl]oxy}ethyl)-2-{2-[4-(hydroxyethyl)phenyl]-2-oxoethyl}-4 oxoazetidin-1-yl)(triphenylphosphoranilidene)acetate (1.6 g) in THF (30ml) were added at room temperature carbon tetrabromide (0.50g, 1.5mmol) and triphenylphosphine (1.2 g, 4.5 mmol), followed by stirring for 2 hours. Thereto were further added at room temperature carbon tetrabromide (0.50 g, 1.5 mmol) and triphenylphosphine (1.2 g, 4.5 mmol), and the mixture was stirred for 11 hours. To the reaction mixture was added a saturated aqueous sodium hydrogen carbonate solution, and the mixture was extracted with ethyl acetate and separated by a separating funnel. The organic layer was washed with saturated brine (twice), dried over anhydrous sodium sulfate, filtered and concentrated. The residue was dissolved in DMF (30 ml) and thereto was added at room temperature morpholine (1.1 g, 22 mmol), followed by stirring for 7 hours. To the reaction mixture was added a saturated aqueous sodium hydrogen carbonate solution, and the mixture was extracted with ethyl acetate and separated by a separating funnel. The organic layer was washed three times with saturated brine, dried over anhydrous sodium sulfate, filtered and concentrated to give allyl ((2R,3S)-3-((1R)-1-{[tert-butyl(dimethyl)silyl]oxy}ethyl)-2-{2-[4-(morpholin-4-ylmethyl)phenyl]-2-oxoethyl}-4-oxoazetidin-1-yl)(triphenylphosphoranilidene)acetate (1.0 g, yield 57%).
LC/MS (EI) 805.37 (M+1)

### Step b)

Allyl ((2R,3S)-3-((1R)-1-{[tert-butyl(dimethyl)silyl]oxy}ethyl)-2-{2-[4-(morpholin-4-ylmethyl)phenyl]-2-oxoethyl}-4-oxoazetidin-1-yl)(triphenylphosphoranilidene)acetate (1.0 g) obtained by step a) was dissolved at room temperature in an aqueous 70% trifluoroacetic acid solution (25 ml). To the reaction mixture was added ethyl acetate (40 ml) and the mixture was washed with saturated brine (70 ml) and a saturated aqueous sodium hydrogen carbonate solution (70 ml), successively, dried over anhydrous sodium sulfate, filtered and concentrated. The residue and triethylamine (0.89ml, 6.4 mmol) were dissolved in THE (40 ml) and thereto was added at 0°C chlorotrimethylsilane (0.57 ml, 4.5 mmol), followed by stirring 10 minutes. Thereto were added at 0°C triethylamine (0.45ml, 3.2 mmol) and chlorotrimethylsilane (0.57ml, 4.5 mmol), followed by stirring for 15 minutes. To the reaction mixture was added ethyl acetate, and the mixture was washed with a saturated aqueous sodium hydrogen carbonate solution (50 ml) and saturated brine (50 mlx 2), successively, dried over anhydrous sodium sulfate, filtered and concentrated to give allyl ((2R,3S)-2-{2-[4-(morpholin-4-ylmethyl)phenyl]-2-oxoethyl}-4-oxo-3-{(1R)-1-[(trimethylsilyl)oxy]ethyl}azetidin-1-yl)(triphenylphosphoranilidene)acetate (1.1 g, yield 100%).
LC/MS (EI) 763.33 (M+1)

### Reference example 15

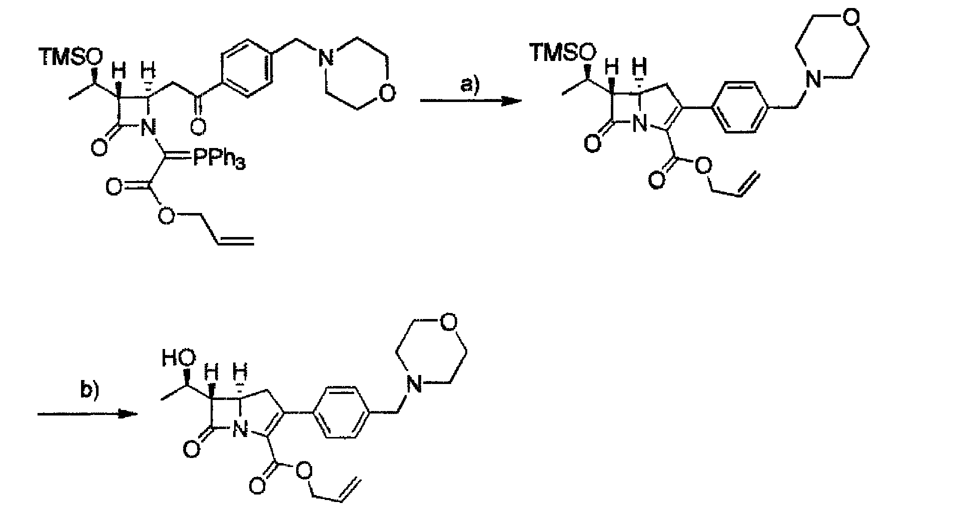

### Step a)

Allyl ((2R,3S)-2-{2-[4-(morpholin-4-ylmethyl)phenyl]-2-oxoethyl}-4-oxo-3-{(1R)-1-[(trimethylsilyl)oxy]ethyl}azetidin-1-yl)(triphenylphosphoranilidene)acetate (1.1 g) obtained by reference example 14 was dissolved in toluene (70 ml) and thereto were added N,O-bistrimethylsilylacetamide (0.63 ml) and hydroquinone (45 mg), followed by refluxing under heating for 9 hours. After being cooled, the reaction mixture was concentrated and the residue was purified by silica gel column chromatography (silica gel 70 g, ethyl acetate: hexane = 1:1 ~ 4:1) to give allyl (5R,6S)-3-[4-(morpholin-4-ylmethyl)phenyl]-7-oxo-6-{(1R)-1-[(trimethylsilyl)oxy]ethyl}-1-azabicyclo[3.2.0]hept-2-ene-2-carboxylate (0.62 g, yield 100%).
¹H NMR (400 MHz, CDCl₃) δ 0.14 (s, 9 H), 1.29 (d, 3 H, J = 6.2 Hz), 2.46 (br. s, 4 H), 3.12-3.29 (m, 3 H), 3.51 (br. s, 2 H), 3.72 (br. s, 4 H), 4.17-4.26 (m, 2 H), 4.60-4.79 (m, 4 H), 5.20 (d, 1 H, J = 10.5 Hz), 5.33 (d, 1 H, J = 17.2 Hz), 5.82-5.96 (m, 1 H), 6.91 (d, 2 H, J = 8.9 Hz), 7.37 (d, 2 H, J = 8.9 Hz).

### Step b)

Allyl (5R,6S)-3-[4-(2-morpholin-4-yl-2-oxoethoxy)phenyl]-7-oxo-6-{(1R)-1-[(trimethylsilyl)oxy]ethyl}-1-azabicyclo[3.2.0]hept-2-ene-2-carboxylate (0.70 g, 1.3 mmol) obtained by step a) was dissolved in THF (45 ml) and water (5 ml), and to the solution was gradually dropped 0.1 N hydrochloric acid under cooling in an ice bath with a pH meter to adjust pH to 3.0. After 30 minutes, thereto were gradually dropped a saturated aqueous sodium hydrogen carbonate solution (40 ml) and saturated brine (40 ml). The mixture was extracted with ethyl acetate (50 ml). The organic layer was dried over anhydrous sodium sulfate, filtered and concentrated to give allyl (5R,6S)-6-[(1R)-1-hydroxyethyl]-3-[4-(2-morpholin-4-yl-2-oxoethoxy)phenyl]-7-oxo-1-azabicyclo[3.2.0]hept-2-ene-2-carboxylate (0.59 g, 1.3 mmol, yield 97%).
¹H NMR (400 MHz, CDCl₃) δ 1.37 (d, 3 H, J = 6.3 Hz), 1.84 (d, 1 H, J = 4.7 Hz), 2.48 (br. s, 4 H), 3.12-3.33 (m, 3 H), 3.56 (br. s, 2 H), 3.73 (br. s, 4 H), 4.20-4.32 (m, 2 H), 4.58-4.78 (m, 2 H), 5.17 (d, 1 H, J = 10.5 Hz), 5.27 (d, 1 H, J = 17.2 Hz), 5.79-5.92 (m, 1 H), 7.35 (br. s, 4 H).

### Reference example 16

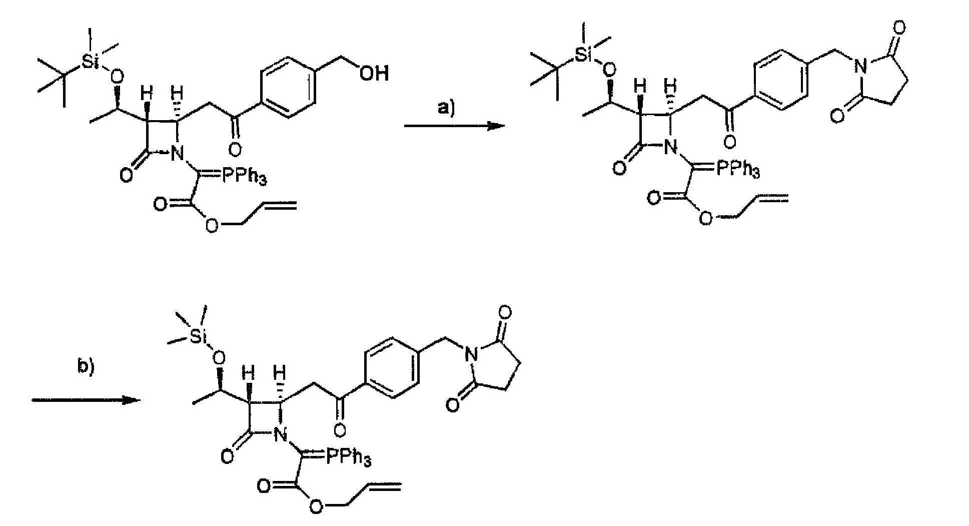

### Step a)

Allyl [(2R,3S)-3-((1R)-1-{[tert-butyl(dimethyl)silyl]oxy}ethyl)-2-(4-hydroxymethylphenyl-2-oxoethyl)-4-oxoazetidin-1-yl](triphenylphosphoranilidene)acetate (1.5 g, 2.03 mmol) was dissolved in methylene chloride (15 ml), and thereto was dropped at room temperature N,N-dimethyl-2-chloropropenylamine (0.32 ml, 2.64 mmol), followed by stirring for 30 minutes. There was obtained a yellow oil by removing the solvent in vacuo. The oil was dissolved in dried DMF (10 ml) and thereto were added and stirred succinimide (1.0 g, 10.2 mmol), tetrabutylammonium bromide (1.3g, 4.08mmol) and cesium carbonate (1.99 g, 6.12 mmol), successively. Two hours later, the reaction mixture was poured into a saturated aqueous ammonium chloride, extracted with ethyl acetate and separated by a separating funnel. The organic layer was washed with water and saturated brine, successively and dried over anhydrous sodium sulfate. The solvent was removed in vacuo to give allyl [(2R,3S)-3-((1R)-1-{[tert-butyl(dimethyl)silyl]oxy}ethyl)-2-(2-{4-[(2,5-dioxopyrrolidin-1-yl)methyl]phenyl}-2-oxoethyl)-4-oxoazetidin-1-yl](triphenylphosphoranilidene)acetate (quantatively, 1.66 g). This product was used for next reaction without further purification.
LC/MS(EI) 712(M+1)

### Step b)

Allyl [(2R,3S)-3-((1R)-1-{[tert-hutyl(dimethyl)silyl]oxy}ethyl)-2-(2-{4-[(2,5-dioxopyrrolidin-1-yl)methyl]phenyl}-2-oxoethyl)-4-oxoazetidin-1-yl](triphenylphosphoranilidene)acetate (1.66g) obtained by step a) was dissolved in a cooled 70% aqueous TFA solution (15 ml). After stirring for 5 minutes the ice bath was removed. The solution was stirred at room temperature for 30 minutes and then again cooled in an ice bath. The reaction mixture was adjusted to pH8 with a saturated aqueous sodium hydrogen carbonate solution, and thereto was added ethyl acetate. The mixture was washed with saturated brine, dried over anhydrous sodium sulfate, filtered and concentrated to give a yellow amorphous (1.67 g). This product was dissolved in THF (27ml) and triethylamine (1.66 ml, 11.8 mmol), and thereto was added at 0°C chlorotrimethylsilane (1.52 ml, 8.4 mmol). After stirring for 5 minutes, the ice bath was removed, and the mixture was stirred at room temperature for 25 minutes. Then the mixture was again cooled in an ice bath. To the reaction mixture were added ethyl acetate and a saturated aqueous sodium hydrogen carbonate solution. The organic layer was washed with saturated brine, dried over sodium sulfate, filtered and concentrated to give allyl ((2R,3S)-2-(2-{4-[(2,5-dioxopyrrolidin-1-yl)methyl]phenyl}-2-oxoethyl)-4-oxo-3-{(1R)-1-[(trimethylsilyl)oxy]ethyl}azetidin-1-yl)(triphenylphosphoranilidene)acetate (quantatively 1.58 g) as a yellow oil. This product was used for next reaction without further purification.
LC/MS(EI) 775(M+1)

### Reference example 17

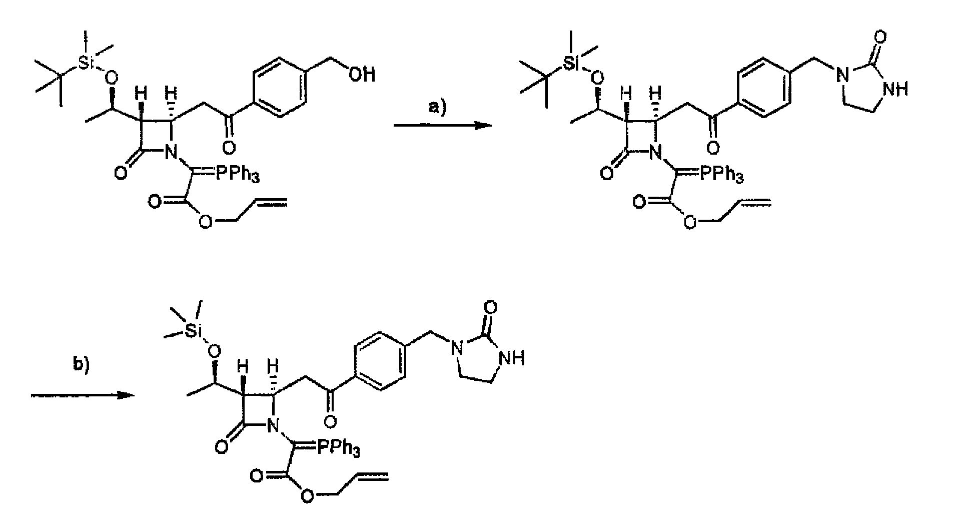

### Step a)

Allyl [(2R,3S)-3-((1R)-1-{[tert-butyl(dimethyl)silyl]oxy}ethyl)-2-(4-hydroxymethylphenyl-2-oxoethyl)-4-oxoazetidin-1-yl](triphenylphosphoranilidene)acetate (1.5g, 2.03mmol) was dissolved in methylene chloride (15 ml), and thereto was dropped at room temperature N,N-dimethyl-2-chloropropenylamine (0.32 ml, 2.64 mmol), followed by stirring for 30 minutes. There was obtained a yellow oil by removing the solvent in vacuo. The oil was dissolved in dried DMF (15 ml), and thereto were added ethylenediamine (0.68 ml, 10.2 mmol) and tetrabutylammonium bromide (1.97 g, 6.12 mmol), successively. After stirring at room temperature overnight, the reaction mixture was poured into a phosphate buffer (pH7.46) under ice cooling, extracted with ethyl acetate and separated by a separating funnel. The organic layer was washed with water and saturated brine, successively, dried over anhydrous sodium sulfate. The solvent was removed in vacuo to give a pale yellow amorphous (1.37 g, yield 88%). This product was dissolved in methylene chloride (28ml) under ice cooling, and thereto was added N,N-dicarbonylimidazole (285 mg, 1.76 mmol) in one portion, and the mixture was gradually warmed to room temperature, followed by stirring overnight. The reaction mixture was poured to 2N aqueous ammonium chloride solution. The mixture was extracted with ethyl acetate and separated by a separating funnel. The organic layer was washed with water and saturated brine, successively, dried over anhydrous sodium sulfate. The solvent was removed in vacuo and the residue was purified by silica gel column chromatography (silica gel 120 ml, chloroform: methanol = 100:0 - 100:5) to give allyl [(3S,4R)-3-((1R)-1-{[tert-butyl(dimethyl)silyl]oxy}ethyl)-2-oxo-4-(2-oxo-2-{4-[(2-oxo-imidazolidin-1-yl)methyl]phenyl}ethyl)azetidin-1-yl](triphenylphosphoranilidene)acetate (0.86 g, yield 59%) as a yellowish white amorphous. This product was used for next reaction without further purification.
LC/MS(EI) 805(M+1)

### Step b)

Allyl [(3S,4R)-3-((1R)-1-{[tert-butyl(dimethyl)silyl]oxy}ethyl)-2-oxo-4-(2-oxo-2-{4-[(2-oxoimidazolidin-1-yl)methyl]phenyl}ethyl)azetidin-1-yl](triphenylphosphoranilidene)acetate (0.83 g, 1.03 mmol) obtained by step a) was dissolved in a cooled. 70% aqueous TFA solution (12 ml). After stirring for 5 minutes the ice bath was removed. The solution was stirred at room temperature for 20 minutes and then again cooled in an ice bath. The reaction mixture was adjusted to pH8 with a saturated aqueous sodium hydrogen carbonate solution, and thereto was added ethyl acetate. The mixture was washed with saturated brine, dried over anhydrous sodium sulfate, filtered and concentrated to give a yellow amorphous. This product was dissolved in THF (8.3ml) and triethylamine (0.72 ml, 5.15 mmol), and thereto was added at 0°C chlorotrimethylsilane (0.46 ml., 3.6 mmol). After stirring for 5 minutes, the ice bath was removed, and the mixture was stirred at room temperature for 20 minutes. Then the mixture was again cooled in an ice bath. To the reaction mixture were added ethyl acetate and a saturated aqueous sodium hydrogen carbonate solution. After extracting and separating by a separating funnel, the organic layer was washed with saturated brine, dried over anhydrous sodium sulfate, filtered and concentrated to give allyl ((3S,4R)-2-oxo-4-[2-oxo-2-(4-{[2-oxo-3-(trimethylsilyl)imidazolidin-1-yl]methyl}phenyl)ethyl]-3-{(1R)-1-[(trimethylsilyl)oxy]ethyl}azetidin-1-yl)(triphenylphosphoranilidene)acetate (0.76 g, yield 97%) as a yellow oil. This product was used for next reaction without further purification.
LC/MS(EI) 762 (M+1)

### Reference example 18

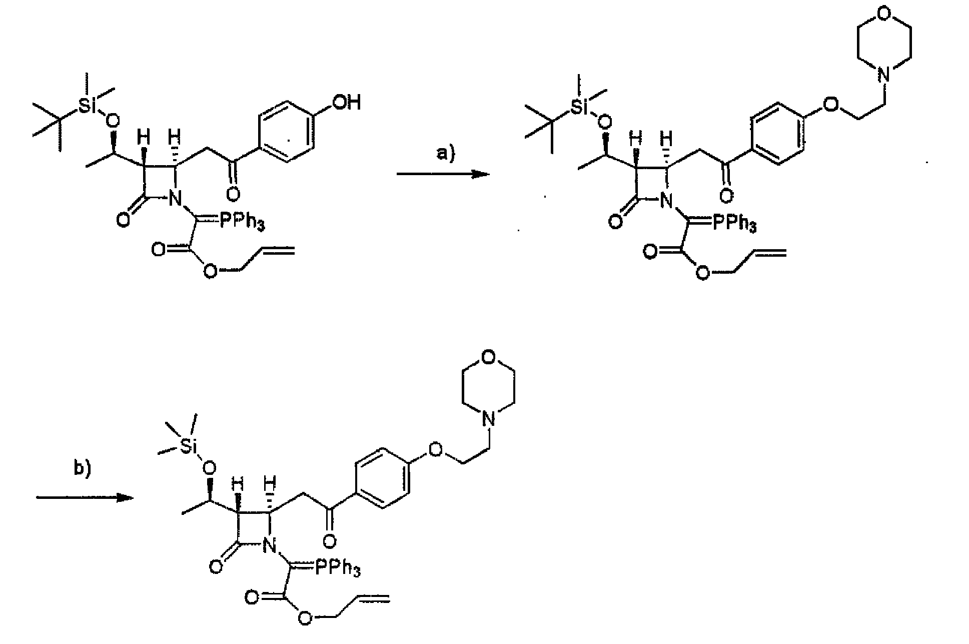

### Step a)

Allyl [(2R,3S)-3-((1R)-1-{[tert-butyl(dimethyl)silyl]oxy}ethyl)-2-(4-hydroxyphenyl)-2-oxoethyl]-4-oxoazetidin-1-yl](triphenylphosphoranilidene)acetate (1.5 g, 2.08 mmol) was dissolved in DMF (30 ml), and thereto were added at room temperature N-2-chloroethylmorpholin (0.8N toluene solution, 6.24 mmol), tetrabutylammonium iodide (2.53 g, 6.86 mmol) and potassium carbonate (0.57 g, 4.16 mmol), successively. After stirring at 50°C for 5 hours, the mixture was allowed to cool. The reaction mixture was poured into a 5% potassium hydrogen sulfate solution under ice cooling, extracted with ethyl acetate and separated by a separating funnel. The organic layer was washed with water and saturated brine, successively and dried over anhydrous sodium sulfate. The solvent was removed in vacuo to give allyl ((2R,3S)-3-((1R)-1-{[tert-butyl(dimethyl)silyl]oxy}ethyl)-2-{2-[4-(2-morpholin-4-ylethoxy)phenyl]-2-oxoethyl}-4-oxoazetidin-1-yl)(triphenylphosphoranilidene)acetate(1.57 g, yield 90%) as a pink amorphous. This product was used for next reaction without further purification.
LC/MS(EI) 836 (M+1)

### Step b)

Allyl ((2R,3S)-3-((1R)-1-{[tert-butyl(dimethyl)silyl]oxy}ethyl)-2-{2-[4-(2-morpholin-4-ylethoxy)phenyl]-2-oxoethyl}-4-oxoazetidin-1-yl)(triphenylphosphoranilidene)acetate (1.57 g, 1.88 mmol) obtained by step a) was dissolved in a cooled 70% aqueous TFA solution (15 ml). After stirring for 5 minutes the ice bath was removed. The solution was stirred at room temperature for 15 minutes and then again cooled in an ice bath. The reaction mixture was adjusted to pH8 with a saturated aqueous sodium hydrogen carbonate solution, and thereto was added ethyl acetate. The mixture was extracted and separated by a separating funnel. The organic layer was washed with saturated brine, dried over anhydrous sodium sulfate, filtered and concentrated to give a pink amorphous. This product was dissolved in THF (27 ml) and triethylamine (1.31 ml, 9.4 mmol), and thereto was added at 0°C chlorotrimethylsilane (1.3 ml, 6.6 mmol). After stirring for 5 minutes, the ice bath was removed, and the mixture was stirred at room temperature for 50 minutes. Then the mixture was cooled in an ice bath. To the reaction mixture were added ethyl acetate and a saturated aqueous sodium hydrogen carbonate solution, and the mixture was extracted and separated by a separating funnel. The organic layer was washed with saturated brine, dried over sodium sulfate, filtered and concentrated to give allyl ((2R,3S)-2-{2-[4-(2-morpholin-4-ylethoxy)phenyl]-2-oxoethyl}-4-oxo-3-{(1R)-1-[(trimethylsilyl)oxy]ethyl}azetidin-1-yl)(triphenylphosphoranilidene)acetate (1.36 g, yield 91%) as a pink amorphous. This product was used for next reaction without further purification.
LC / MS(EI) 792 (M+1)

### INDUSTRIAL APPLICABILITY

The novel carbapenem compound of the present invention can be used as a potent and safe antibacterial agent for oral administration having a broad spectrum and excellent antibacterial activity against Gram positive bacteria and Gram negative bacteria, especially Haemophilus influenzae which obtain resistance to the inhibitory effect of existing β-lactam agents together with mutation of a penicillin binding proteins (PBP) such as β-lactamase non-producing ampicillin resistant Haemophilus influenzae (BLNAR), and penicillin resistant Streptococcus pneumoniae (PRSP) which are recently increasingly isolated and provide a clinical problem.

## Claims

1. A carbapenem compound represented by the formula [1]: 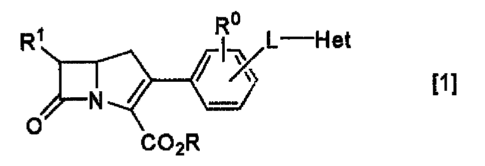 wherein R⁰ is hydrogen atom, C₁ to C₄ alkyl, C₁ to C₄ alkoxy, trifluoromethoxy, halogen atom or cyano group,
R¹ is C₁ to C₃ alkyl or C₁ to C₃ alkyl substituted by hydroxy,
R is hydrogen atom or a group which regenerates a carboxyl group by hydrolysis in a living body,
L is a single bond, methylene, ethylene, propylene, -O(CH₂)₂- or -OCH₂(CO)-, and
Het is a monocyclic heteroaromatic ring having a nitrogen atom(s), or a group represented by the formula [2]: 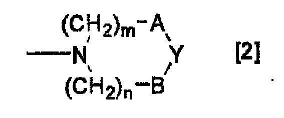 wherein m and n are independently 0 or 1,
A and B are independently methylene, carbonyl or thiocarbonyl,
Y is methylene, ethylene, oxygen, -OCH₂-, sulfur, -SCH₂-, -NR^{a}- or-NR^{a}CH₂-(wherein R^{a} is hydrogen atom, an amine protective group or an optionally substituted C₁ to C₄ alkyl group), provided that when Y is methylene, either A or B is at least carbonyl or thiocarbonyl, and when m and n are 0, and A and B are independently carbonyl or thiocarbonyl, Y is methylene, ethylene or propylene,
or its pharmaceutically acceptable salt.

2. The carbapenem compound according to claim 1 wherein the group which regenerates a carboxyl group by hydrolysis in a living body is a group of the formula [3]: 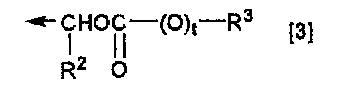 wherein R² is hydrogen atom or C₁ to C₆ alkyl group, R³ is an optionally substituted C₁ to C₁₀ alkyl group, or an optionally substituted C₃ to C₁₀ cycloalkyl group, and t is 0 or 1,
or (5-t-butyl-2-oxo-1,3-dioxol-4-yl)methyl,
or its pharmaceutically acceptable salt.

3. The carbapenem compound according to claim 1 wherein R is a group represented by the formula [3]: 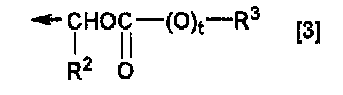 wherein R², R³ and t are the same as in claim 2,
or (5-t-butyl-2-oxo-1,3-dioxol-4-yl)methyl,
or its pharmaceutically acceptable salt.

4. The carbapenem compound according to any one of claims 1 to 3 wherein R¹ is 1-hydroxyethyl, or its pharmaceutically acceptable salt.

5. The carbapenem compound according to any one of claim 1 to 4 wherein Het is morpholino, or its pharmaceutically acceptable salt.

6. The carbapenem compound according to any one of claims 1 to 4 wherein Het is a group represented by the formula [4]: 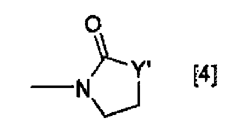 wherein Y' is methylene, ethylene, oxygen, -OCH₂-, -CH₂O-, sulfur, -SCH₂-, -CH₂S-, -NR^{a}-, -NR^{a}CH₂- or -CH₂NR^{a}- (wherein R^{a} is hydrogen atom, amino protecting group or optionally substituted C₁ to C₄ alkyl group), or its pharmaceutically acceptable salt.

7. The carbapenem compound according to any one of claims 1 to 6 wherein L is a single bond, ethylene, propylene or -O(CH₂)₂-, or its pharmaceutically acceptable salt.

8. The carbapenem compound according to any one of claims 1 to 6 wherein L is methylene, or its pharmaceutically acceptable salt.

9. The carbapenem compound according to any one of claims 1 to 3 wherein R is pivaloyloxymethyl or (5-t-butyl-2-oxo-1,3-dioxol-4-yl)methyl, or its pharmaceutically acceptable salt.

10. The carbapenem compound according to any one of claims 1 to 9 wherein Het is a monocyclic heteroaromatic ring containing a nitrogen atom (s), or its pharmaceutically acceptable salt.

11. A medicament containing the carbapenem compound or its pharmaceutically acceptable salt according to any one of claims 1 to 10 as an active ingredient.

12. An antibacterial agent for oral administration containing the carbapenem compound or its pharmaceutically acceptable salt according to any one of claims 1 to 10 as an active ingredient.
